(19) Europäisches Patentamt
European Patent Office
Office européen des brevets

(11) EP 1 967 242 A1

(12) EUROPEAN PATENT APPLICATION
published in accordance with Art. 158(3) EPC

(43) Date of publication:
10.09.2008 Bulletin 2008/37

(21) Application number: 06842908.3

(22) Date of filing: 20.12.2006

(51) Int Cl.:
*B01D 3/14* (2006.01) *B01D 3/22* (2006.01)
*B01D 3/32* (2006.01) *C07B 61/00* (2006.01)
*C07C 27/02* (2006.01) *C07C 29/128* (2006.01)
*C07C 31/20* (2006.01) *C07C 68/06* (2006.01)
*C07C 68/08* (2006.01) *C07C 69/96* (2006.01)

(86) International application number:
PCT/JP2006/325354

(87) International publication number:
WO 2007/074692 (05.07.2007 Gazette 2007/27)

(84) Designated Contracting States:
AT BE BG CH CY CZ DE DK EE ES FI FR GB GR HU IE IS IT LI LT LU LV MC NL PL PT RO SE SI SK TR

(30) Priority: 27.12.2005 JP 2005374731

(71) Applicant: Asahi Kasei Chemicals Corporation
Tokyo 100-8440 (JP)

(72) Inventors:
- FUKUOKA, Shinsuke
Tokyo 100-8440 (JP)
- MIYAJI, Hironori
Tokyo 100-8440 (JP)
- HACHIYA, Hiroshi
Tokyo 100-8440 (JP)
- MATSUZAKI, Kazuhiko
Tokyo 100-8440 (JP)

(74) Representative: Strehl Schübel-Hopf & Partner
Maximilianstrasse 54
80538 München (DE)

# (54) PROCESS FOR INDUSTRIAL PRODUCTION OF DIALKYL CARBONATE AND DIOL

(57) It is an object of the present invention, when producing a dialkyl carbonate and a diol industrially in a large amount (e.g. not less than 2 ton / hr for the dialkyl carbonate and not less than 1.3 ton / hr for the diol) through a reactive distillation system of taking a cyclic carbonate and an aliphatic monohydric alcohol as starting materials, continuously feeding the starting materials into a continuous multi-stage distillation column in which a catalyst is present, and carrying out reaction and distillation simultaneously in the column, is to provide a specific process that enables the dialkyl carbonate and the diol to be produced each with high selectivity and high productivity stably for a prolonged period of time (e.g. not less than 1000 hours, preferably not less than 3000 hours, more preferably not less than 5000 hours), and moreover enables the dialkyl carbonate produced to be separated out and purified stably for a prolonged period of time with high efficiency . It has been discovered that by carrying out reactive distillation using a continuous multi-stage distillation column A having a specified structure, and subjecting a column top component $A_T$ thus obtained to separation by distillation using a continuous multi-stage distillation column B having a specified structure, a dialkyl carbonate of high purity (e.g. not less than 97%, preferably not less than 99%, more preferably not less than 99.9%, yet more preferably not less than 99.99%) and a diol can be produced each with a high selectivity of not less than 95%, preferably not less than 97%, more preferably not less than 99%, on an industrial scale of not less than 2 ton / hr, preferably not less than 3 ton / hr, more preferably not less than 4 ton / hr, for the dialkyl carbonate, and not less than 1.3 ton / hr, preferably not less than 1.95 ton / hr, more preferably not less than 2.6 ton / hr, for the diol, with a high yield stably for a prolonged period of time of not less than 1000 hours, preferably not less than 3000 hours, more preferably not less than 5000 hours, from a cyclic carbonate and an aliphatic monohydric alcohol.

EP 1 967 242 A1

FIG.1

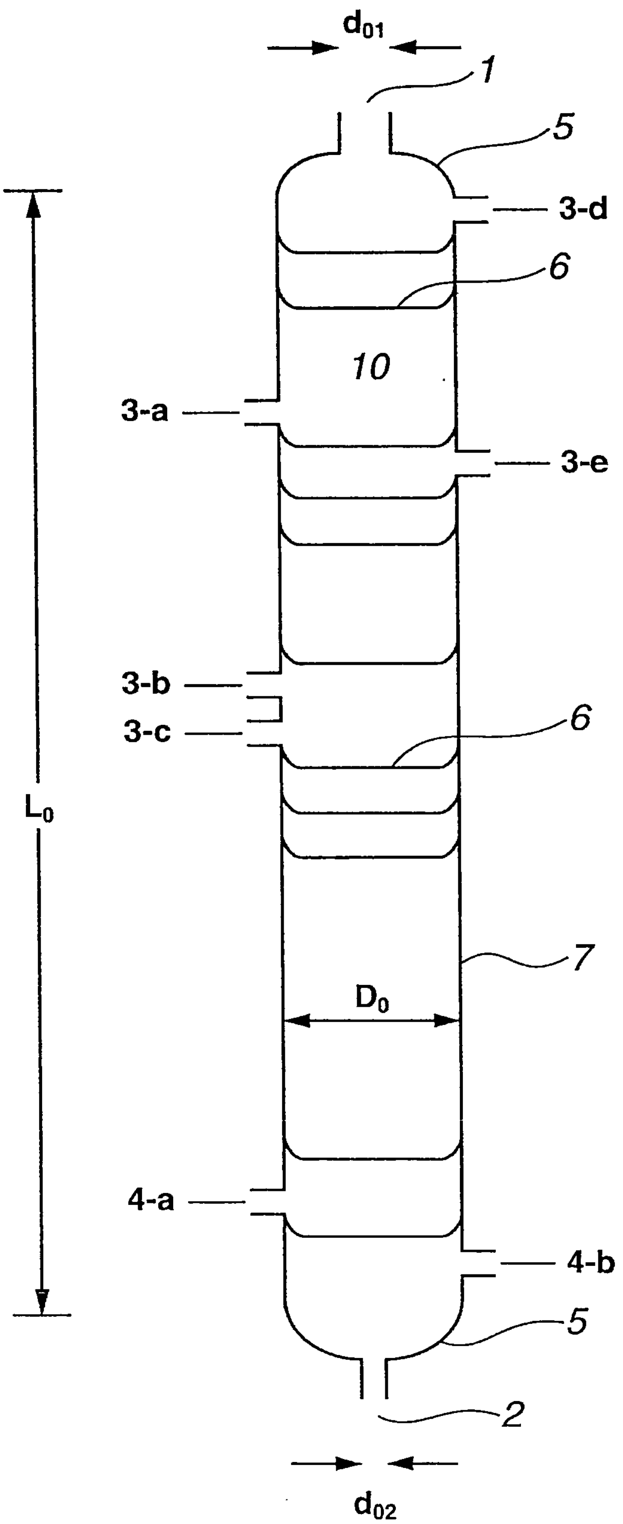

## Description

Technical Field

The present invention relates to an industrial process for the production of a dialkyl carbonate and a diol. More particularly, the present invention relates to a process for industrially producing large amounts of the dialkyl carbonate and the diol stably for a prolonged period of time by taking a cyclic carbonate and an aliphatic monohydric alcohol as starting materials, continuously feeding the starting materials into a continuous multi-stage distillation column in which a catalyst is present, carrying out reactive distillation in the column so as to obtain a high boiling point reaction mixture having the diol as a main component thereof from a lower portion of the column and continuously withdraw a low boiling point reaction mixture containing the dialkyl carbonate from an upper portion of the column, continuously feeding the low boiling point reaction mixture into another continuous multi-stage distillation column, and carrying out purification by distillation so as to obtain the dialkyl carbonate.

Background Art

Several processes for the production of a dialkyl carbonate and a diol from a reaction between a cyclic carbonate and an aliphatic monohydric alcohol have been proposed, but most of these proposals relate to a catalyst. As reaction systems, four systems have been proposed hitherto. These four reaction systems are used in a process for the production of dimethyl carbonate and ethylene glycol from ethylene carbonate and methanol, which is the most typical reaction example.

A first system is a completely batch reaction system in which ethylene carbonate, methanol and a catalyst are put into an autoclave, which is a batch reaction vessel, and reaction is carried out by holding for a predetermined reaction time under an applied temperature at a reaction temperature above the boiling point of methanol (see, for example, Patent Document 1: U.S. Patent No. 3642858, Patent Document 2: Japanese Patent Application Laid-Open No. S54-48715 (corresponding to U.S. Patent No. 4181676), Patent Document 5: Japanese Patent Application Laid-Open No.S54-63023, Patent Document 6: Japanese Patent Application Laid-Open No. S54-148726).

A second system is a system that uses an apparatus in which a distillation column is provided on top of a reaction vessel; ethylene carbonate, methanol and a catalyst are put into the reaction vessel, and reaction is made to proceed by heating to a predetermined temperature. With this system, to make up for methanol distilled off through azeotropy with the produced dimethyl carbonate, methanol is added to the reaction vessel continuously or in batches, but in any event with this system the reaction proceeds only in the reaction vessel, which is batch type, in which the catalyst, ethylene carbonate and methanol are present. The reaction is thus batch type, the reaction being carried out using a large excess of methanol under reflux taking a long time in a range of from 3 to over 20 hours (see, for example, Patent Document 3: Japanese Patent Application Laid-Open No. S51-122025 (corresponding to U.S. Patent No. 4062884), Patent Document 4: Japanese Patent Application Laid-Open No. S54-48716 (corresponding to U.S. Patent No. 4307032), Patent Document 11: U.S. Patent No. 3803201).

A third system is a continuous reaction system in which a mixed solution of ethylene carbonate and methanol is continuously fed into a tubular reactor maintained at a predetermined reaction temperature, and a reaction mixture containing unreacted ethylene carbonate and methanol and product dimethyl carbonate and ethylene glycol is continuously withdrawn in a liquid form from an outlet on the other side. Either of two processes is used depending on the form of the catalyst used. That is, there are a process in which a homogeneous catalyst is used, and is passed through the tubular reactor together with the mixed solution of ethylene carbonate and methanol, and then after the reaction the catalyst is separated out from the reaction mixture (see, for example, Patent Document 7: Japanese Patent Application Laid-Open No. 63-41432 (corresponding to U.S. Patent No. 4661609), Patent Document 10: U.S. Patent No. 4734518), and a process in which a heterogeneous catalyst fixed inside the tubular reactor is used (see, for example, Patent Document 8: Japanese Patent Application Laid-Open No. S63-238043, Patent Document 9: Japanese Patent Application Laid-Open No. S64-31737 (corresponding to U.S. Patent No. 4691041)). The reaction producing dimethyl carbonate and ethylene glycol through reaction between ethylene carbonate and methanol is an equilibrium reaction, and hence with this continuous flow reaction system using a tubular reactor, it is impossible to make the ethylene carbonate conversion higher than the equilibrium conversion determined by the composition ratio put in and the reaction temperature. For example, according to Example 1 in Patent Document 7 (Japanese Patent Application Laid-Open No. S63-41432 (corresponding to U.S. Patent No. 4661609)), for a flow reaction at 130°C using a starting material put in with a molar ratio of methanol / ethylene carbonate = 4 / 1, the ethylene carbonate conversion is 25%. This means that a large amount of unreacted ethylene carbonate and methanol remaining in the reaction mixture must be separated out, recovered, and recirculated back into the reactor, and in actual fact, with the process of Patent Document 9 (Japanese Patent Application Laid-Open No. S64-31737 (corresponding to U.S. Patent No. 4691041)), much equipment is used for such separation, purification, recovery, and recirculation.

A fourth system is a reactive distillation system first disclosed by the present inventors (see, for example, Patent Document 12: Japanese Patent Application Laid-Open No. H4-198141, Patent Document 13: Japanese Patent Application Laid-Open No. H4-230243, Patent Document 14: Japanese Patent Application Laid-Open No. H9-176061, Patent Document 15: Japanese Patent Application Laid-Open No. H9-183744, Patent Document 16: Japanese Patent Application Laid-Open No. H9-194435, Patent Document 17: International Publication No. WO97/23445 (corresponding to European Patent No. 0889025, U.S. Patent No. 5847189), Patent Document 18: International Publication No. WO99/64382 (corresponding to European Patent No. 1086940, U.S. Patent No. 6346638), Patent Document 19: International Publication No. WO00/51954 (corresponding to European Patent No. 1174406, U.S. Patent No. 6479689), Patent Document 20: Japanese Patent Application Laid-Open No. 2002-308804, Patent Document 21: Japanese Patent Application Laid-Open No. 2004-131394), that is a continuous production process in which ethylene carbonate and methanol are each continuously fed into a multi-stage distillation column, and reaction is carried out in the presence of a catalyst in a plurality of stages in the distillation column, while the dimethyl carbonate and ethylene glycol which are produced are separated off. Patent applications in which such a reactive distillation system is used have subsequently been filed by other companies (see, for example, Patent Document 22: Japanese Patent Application Laid-Open No. H5-213830 (corresponding to European Patent No. 0530615, U.S. Patent No. 5231212), Patent Document 23: Japanese Patent Application Laid-Open No. H6-9507 (corresponding to European Patent No. 0569812, U.S. Patent No. 5359118), Patent Document 24: Japanese Patent Application Laid-Open No. 2003-119168 (corresponding to International Publication No. WO03/006418), Patent Document 25: Japanese Patent Application Laid-Open No. 2003-300936, Patent Document 26: Japanese Patent Application Laid-Open No 2003-342209).

In this way, the processes proposed hitherto for producing the dialkyl carbonate and the diol from the cyclic carbonate and the aliphatic monohydric alcohol are the four systems:

(1) a completely batch reaction system;
(2) a batch reaction system using a reaction vessel having a distillation column provided on top thereof;
(3) a flowing liquid reaction system using a tubular reactor; and
(4) a reactive distillation system.

However, there have been problems with these as follows.

In the case of (1) and (3), the upper limit of the cyclic carbonate conversion is determined by the composition put in and the temperature, and hence the reaction cannot be carried out to completion, and thus the conversion is low. Moreover, in the case of (2), to make the cyclic carbonate conversion high, the produced dialkyl carbonate must be distilled off using a very large amount of the aliphatic monohydric alcohol, and a long reaction time is required. In the case of (4), the reaction can be made to proceed with a higher conversion than with (1), (2) or (3). However, processes of (4) proposed hitherto have related to producing the dialkyl carbonate and the diol either in small amounts or for a short period of time, and have not related to carrying out the production on an industrial scale stably for a prolonged period of time. That is, these processes have not attained the object of producing a dialkyl carbonate continuously in a large amount (e.g. not less than 2 ton / hr) stably for a prolonged period of time (e.g. not less than 1000 hours, preferably not less than 3000 hours, more preferably not less than 5000 hours).

For example, the maximum values of the height (H: cm), diameter (D: cm), and number of stages (n) of the reactive distillation column, the produced amount P (kg / hr) of dimethyl carbonate, and the continuous production time T (hr) in examples disclosed for the production of dimethyl carbonate (DMC) and ethylene glycol (EG) from ethylene carbonate and methanol are as in Table 1. Table 1

**TABLE 1**

| PATENT DOCUMENT | H:cm | D:cm | NO. STAGES: n | P : kg/hr | T : hr |
|---|---|---|---|---|---|
| 12 | 100 | 2 | 30 | 0.106 | 400 |
| 15 | 160 | 5 | 40 | 0.427 | NOTE 5 |
| 16 | 160 | 5 | 40 | 0.473 | NOTE 5 |
| 18 | 200 | 4 | PACKING COLUMN (Dixon) | 0.932 | NOTE 5 |
| 19 | NOTE 1 | 5 | 60 | 0.275 | NOTE 5 |
| 20 | NOTE 1 | 5 | 60 | 0.258 | NOTE 5 |
| 21 | NOTE 1 | 5 | 60 | 0.258 | NOTE 5 |
| 22 | 250 | 3 | PACKING COLUMN (Raschig) | 0.392 | NOTE 5 |

(continued)

| PATENT DOCUMENT | H:cm | D:cm | NO. STAGES: n | P : kg/hr | T : hr |
|---|---|---|---|---|---|
| 23 | NOTE 2 | NOTE 2 | NOTE 2 | 0.532 | NOTE 5 |
| 24 | NOTE 3 | NOTE 3 | 42 | NOTE 4 | NOTE 5 |
| 25 | NOTE 3 | NOTE 3 | 30 | 3750 | NOTE 5 |
| 26 | 200 | 15 | PACKING COLUMN (BX) | 0.313 | NOTE 5 |
| NOTE 1 : OLDERSHAW DISTILLATION COLUMN.<br>NOTE 2 : NO DESCRIPTION WHATSOEVER DEFINING DISTILLATION COLUMN.<br>NOTE 3 : ONLY DESCRIPTION DEFINING DISTILLATION COLUMN IS NUMBER OF STAGES.<br>NOTE 4 : NO DESCRIPTION WHATSOEVER OF PRODUCED AMOUNT.<br>NOTE 5 : NO DESCRIPTION WHATSOEVER REGARDING STABLE PRODUCTION FOR PROLONGED PERIOD OF TIME. | | | | | |

In Patent Document 25 (Japanese Patent Application Laid-Open No. 2003-300936), it is stated at paragraph 0060 "The present example uses the same process flow as for the preferred mode shown in FIG. 1 described above, and was carried out with the object of operating a commercial scale apparatus for producing dimethyl carbonate and ethylene glycol through transesterification by a catalytic conversion reaction between ethylene carbonate and methanol. Note that the following numerical values in the present example can be adequately used in the operation of an actual apparatus", and as that example it is stated that 3750 kg / hr of dimethyl carbonate was specifically produced. The scale described in that example corresponds to an annual production of 30,000 tons or more, and hence this implies that at the time of the filing of the patent application for Patent Document 25 (Japanese Patent Application Laid-Open No. 2003-300936) (April 9, 2002), operation of the world's first large scale commercial plant using this process had been carried out. However, even at the time of filing the present application, there is not the above fact at all. Moreover, in the example of Patent Document 25 (Japanese Patent Application Laid-Open No. 2003-300936), exactly the same value as the theoretically calculated value is stated for the produced amount of dimethyl carbonate, but the yield for ethylene glycol is approximately 85.6%, and the selectivity is approximately 88.4%, and hence it cannot really be said that a high yield and high selectivity have been attained. In particular, the low selectivity indicates that this process has a fatal drawback as an industrial production process. (Note also that Patent Document 25 (Japanese Patent Application Laid-Open No. 2003-300936) was deemed to have been withdrawn on July 26, 2005 due to examination not having been requested).

With the reactive distillation method, there are very many causes of fluctuation such as composition variation due to reaction and composition variation due to distillation in the distillation column, and temperature variation and pressure variation in the column, and hence continuing stable operation for a prolonged period of time is accompanied by many difficulties, and in particular these difficulties are further increased in the case of handling large amounts. To continue mass production of a dialkyl carbonate and a diol using the reactive distillation method stably for a prolonged period of time while maintaining high yields and high selectivities for the dialkyl carbonate and the diol, the reactive distillation apparatus must be cleverly devised. However, the only description of continuous stable production for a prolonged period of time with the reactive distillation method proposed hitherto has been the 200 to 400 hours in Patent Document 12 (Japanese Patent Application Laid-Open No. H4-198141) and Patent Document 13 (Japanese Patent Application Laid-Open No. H4-230243).

There has thus been a need to establish an industrial reactive distillation process that enables a dialkyl carbonate and a diol to be mass-produced continuously and stably for a prolonged period of time with high yield and high selectivity, and at this time to further establish an industrial process for separating out the desired dialkyl carbonate efficiently from a low boiling point reaction mixture continuously withdrawn in a large amount from an upper portion of the reactive distillation column. The present invention has been devised to attain this object.

As shown in Table 1, with the exception of Patent Document 25, the produced amount of the dialkyl carbonate per hour using reactive distillation processes proposed hitherto has been a small amount of not more than 1 kg / hr. Moreover, with the process of Patent Document 25, a column top component (a mixture of methanol and dimethyl carbonate) from a first step reactive distillation column is fed into a second step distillation column, and extractive distillation is carried out using ethylene carbonate. After a mixture of ethylene carbonate and dimethyl carbonate has been obtained as a column bottom component from the second step distillation column, this mixture is then further fed into a third step distillation column, and separation by distillation is carried out so as to obtain dimethyl carbonate as a column top component and ethylene carbonate as a column bottom component from the third step distillation column. That is, with the process of Patent Document 25, two columns must be used to separate the dimethyl carbonate out

from the mixture of methanol and dimethyl carbonate, and hence the equipment is expensive. Furthermore, with this process, four distillation columns must be operated together with one another, and hence it is expected that prolonged stable operation would be difficult.

## Disclosure of Invention

### Problems to be Solved by the Invention

It is an object of the present invention to provide, for the case of producing a dialkyl carbonate and a diol industrially in large amounts (e.g. not less than 2 ton / hr for the dialkyl carbonate, and not less than 1.3 ton/hr for the diol) through a reactive distillation system of taking a cyclic carbonate and an aliphatic monohydric alcohol as starting materials, continuously feeding the starting materials into a continuous multi-stage distillation column in which a catalyst is present, and carrying out reaction and distillation simultaneously in the column, a specific process according to which the dialkyl carbonate and the diol can be produced with high selectivity and high productivity stably for a prolonged period of time (e.g. not less than 1000 hours, preferably not less than 3000 hours, more preferably not less than 5000 hours), and further the produced dialkyl carbonate can be separated out and purified stably for a prolonged period of time with high efficiency.

### Means for Solving the Problems

Since the present inventors first disclosed a process for continuously producing a dialkyl carbonate and a diol using a continuous multi-stage distillation column, there have been many proposals regarding improving this process. However, these have been on a small scale and short operating time laboratory level, and there have been no disclosures whatsoever on a specific process or apparatus enabling mass production and separation / purification on an industrial scale stably for a prolonged period of time based on findings obtained through actual implementation. The present inventors have thus carried out studies aimed at discovering a specific process enabling a dialkyl carbonate and a diol to be produced on an industrial scale of, for example, not less than 2 ton / hr for the dialkyl carbonate and not less than 1.3 ton / hr for the diol stably for a prolonged period of time with high selectivity and high productivity, and further enabling the produced dialkyl carbonate to be separated out and purified stably for a prolonged period of time with high efficiency. As a result, the present inventors have reached to the present invention.

That is, according to the first aspect of the present invention, there are provided:

1. an industrial process for the production of a dialkyl carbonate and a diol in which the dialkyl carbonate and the diol are continuously produced from a cyclic carbonate and an aliphatic monohydric alcohol, comprising the steps of:

(I) continuously producing a dialkyl carbonate and a diol through a reactive distillation system of continuously feeding the cyclic carbonate and the aliphatic monohydric alcohol into a continuous multi-stage distillation column A in which a catalyst is present, carrying out reaction and distillation simultaneously in said column, continuously withdrawing a low boiling point reaction mixture $A_T$ containing the produced dialkyl carbonate and unreacted aliphatic monohydric alcohol from an upper portion of the column A in a gaseous form, and continuously withdrawing a high boiling point reaction mixture $A_B$ containing the diol from a lower portion of the column A in a liquid form; and

(II) continuously feeding the low boiling point reaction mixture $A_T$ containing the dialkyl carbonate and the aliphatic monohydric alcohol continuously withdrawn from the upper portion of said continuous multi-stage distillation column A into a continuous multi-stage distillation column B, and continuously separating and purifying into a column top component $B_T$ having the aliphatic monohydric alcohol as a main component thereof and a column bottom component $B_B$ having the dialkyl carbonate as a main component thereof, wherein:

(a) said continuous multi-stage distillation column A is a tray column having a cylindrical trunk portion having a length $L_0$ (cm) and an inside diameter $D_0$ (cm) and having thereinside a tray with a number of stages no, the tray having a plurality of holes therein, and further having a gas outlet having an inside diameter $d_{01}$ (cm) at a top of the column or in an upper portion of the column near to the top, a liquid outlet having an inside diameter $d_{02}$ (cm) at a bottom of the column or in a lower portion of the column near to the bottom, at least one first inlet provided in the upper portion and / or a middle portion of the column below the gas outlet, and at least one second inlet provided in the middle portion and / or the lower portion of the column above the liquid outlet, wherein $L_0$, $D_0$, $n_0$, $d_{01}$ and $d_{02}$ satisfy the following formulae (1) to (6):

$$2100 \leq L_0 \leq 8000 \quad (1)$$

$$180 \leq D_0 \leq 2000 \quad (2)$$

$$4 \leq L_0 / D_0 \leq 40 \quad (3)$$

$$20 \leq n_0 \leq 120 \quad (4)$$

$$3 \leq D_0 / d_{01} \leq 20 \quad (5)$$

$$5 \leq D_0 / d_{02} \leq 30 \quad (6);$$

and

(b) said continuous multi-stage distillation column B is a distillation column comprising a stripping section having a length $L_1$ (cm), an inside diameter $D_1$ (cm) and an internal with a number of stages $n_1$ thereinside, and an enrichment section having a length $L_2$ (cm), an inside diameter $D_2$ (cm) and an internal with a number of stages $n_2$ thereinside, wherein $L_1$, $D_1$, $n_1$, $L_2$, $D_2$, and $n_2$ satisfy the following formulae (7) to (14):

$$500 \leq L_1 \leq 3000 \quad (7)$$

$$100 \leq D_1 \leq 1000 \quad (8)$$

$$2 \leq L_1 / D_1 \leq 30 \quad (9)$$

$$10 \leq n_1 \leq 40 \quad (10)$$

$$700 \leq L_2 \leq 5000 \quad (11)$$

$$50 \leq D_2 \leq 800 \quad (12)$$

$$10 \leq L_2 / D_2 \leq 50 \quad (13);$$

and

$$35 \leq n_2 \leq 100 \qquad (14).$$

2. the process according to item 1, wherein a produced amount of the dialkyl carbonate is not less than 2 ton / hr,
3. the process according to item 1 or 2, wherein a produced amount of the diol is not less than 1.3 ton / hr.
4. the process according to any one of items 1 to 3, wherein said $d_{01}$ and said $d_{02}$ satisfy the following formula (15);

$$1 \leq d_{01} / d_{02} \leq 5 \qquad (15),$$

5. the process according to any one of items 1 to 4, wherein $L_0$, $D_0$, $L_0 / D_0$, $n_0$, $D_0 / d_{01}$, and $D_0 / d_{02}$ for said continuous multi-stage distillation column A satisfy respectively $2300 \leq L_0 \leq 6000$, $200 \leq D_0 \leq 1000$, $5 \leq L_0 / D_0 \leq 30$, $30 \leq n_0 \leq 100$, $4 \leq D_0 / d_{01} \leq 15$, and $7 \leq D_0 / d_{02} \leq 25$,
6. the process according to any one of items 1 to 5, wherein $L_0$, $D_0$, $L_0 / D_0$, $n_0$, $D_0 / d_{01}$, and $D_0 / d_{02}$ for said continuous multi-stage distillation column A satisfy respectively $2500 \leq L_0 \leq 5000$, $210 \leq D_0 \leq 800$, $7 \leq L_0 / D_0 \leq 20$, $40 \leq n_0 \leq 90$, $5 \leq D_0 / d_{01} \leq 13$, and $9 \leq D_0 / d_{02} \leq 20$,
7. the process according to any one of items 1 to 6, wherein the tray in said continuous multi-stage distillation column A is a sieve tray,
8. the process according to item 7, wherein said sieve tray in said continuous multi-stage distillation column A has 100 to 1000 holes / $m^2$ in a sieve portion thereof,
9. the process according to item 7 or 8, wherein a cross-sectional area per hole of said sieve tray in said continuous multi-stage distillation column A is in a range of from 0.5 to 5 $cm^2$,
10. the process according to any one of items 7 to 9, wherein an aperture ratio of said sieve tray in said continuous multi-stage distillation column A is in a range of from 1.5 to 15%.
11. the process according to any one of items 1 to 10, wherein $L_1$, $D_1$, $L_1 / D_1$, $n_1$, $L_2$, $D_2$, $L_2 / D_2$, and $n_2$ for said continuous multi-stage distillation column B satisfy $800 \leq L_1 \leq 2500$, $120 \leq D_1 \leq 800$, $5 \leq L_1 / D_1 \leq 20$, $13 \leq n_1 \leq 25$, $1500 \leq L_2 \leq 3500$, $70 \leq D_2 \leq 600$, $15 \leq L_2 / D_2 \leq 30$, $40 \leq n_2 \leq 70$, $L_1 \leq L_2$, and $D_2 \leq D_1$,
12. the process according to any one of items 1 to 11, wherein the internal in each of the stripping section and the enrichment section of said continuous multi-stage distillation column B is a tray and / or a packing,
13. the process according to item 12, wherein the internal in each of the stripping section and the enrichment section of said continuous multi-stage distillation column B is the tray,
14. the process according to item 13, wherein said tray in said continuous multi-stage distillation column B is a sieve tray,
15. the process according to item 14, wherein said sieve tray in said continuous multi-stage distillation column B has 150 to 1200 holes / $m^2$ in a sieve portion thereof, and a cross-sectional area per hole in a range of from 0.5 to 5 $cm^2$,
16. the process according to any one of items 1 to 15, wherein a concentration of the dialkyl carbonate in said column bottom component $B_B$ said the continuous multi-stage distillation column B is not less than 97 % by weight based on 100 % by weight of said column bottom component,
17. the process according to any one of items 1 to 15, wherein the concentration of the dialkyl carbonate in said column bottom component $B_B$ from said continuous multi-stage distillation column B is not less than 99 % by weight based on 100 % by weight of said column bottom component,
18. the process according to any one of items 1 to 17, wherein said column top component $B_T$ from said continuous multi-stage distillation column B is recycled as a starting material for producing a dialkyl carbonate and a diol,
19. the process according to any one of items 1 to 18, wherein the cyclic carbonate comprises ethylene carbonate and / or propylene carbonate, the aliphatic monohydric alcohol comprises methanol and / or ethanol, the dialkyl carbonate to be produced comprises dimethyl carbonate and / or diethyl carbonate, and the diol to be produced comprises ethylene glycol and / or propylene glycol.

In addition, according to the second aspect of the present invention, there are provided:

20. a dialkyl carbonate separated out by the process according to any one of items 1 to 19, which comprises a halogen content of not more than 0.1 ppm,
21. a dialkyl carbonate separated out by the process according to any one of items 1 to 19, which comprises a halogen content of not more than 1 ppb,
22. the dialkyl carbonate according to item 20 or 21, which is a high-purity dialkyl carbonate having an aliphatic monohydric alcohol content of not more than 0.1 % by weight.

Advantageous Effects of the Invention

It has been discovered that by implementing the present invention, a dialkyl carbonate and a diol can be produced each with a high selectivity of not less than 95%, preferably not less than 97%, more preferably not less than 99%, on an industrial scale of not less than 2 ton / hr, preferably not less than 3 ton / hr, more preferably not less than 4 ton/hr, for the dialkyl carbonate, and not less than 1.3 ton / hr, preferably not less than 1.95 ton / hr, more preferably not less than 2.6 ton / hr, for the diol, stably for a prolonged period of time of not less than 1000 hours, preferably not less than 3000 hours, more preferably not less than 5000 hours, from a cyclic carbonate and an aliphatic monohydric alcohol, and moreover a dialkyl carbonate of high purity (e.g. not less than 97%, preferably not less than 99%, more preferably not less than 99.9%) can be separated out and purified with high efficiency.

Brief Description of Drawings

FIG. 1 is an example of schematic view showing a continuous multi-stage distillation column A used in step (I) in the present invention, $n_0$ stages of trays (shown schematically in FIG. 1) being installed in a trunk portion thereof; and FIG. 2 is an example of schematic view showing a continuous multi-stage distillation column B used in step (II), $n_1$ and $n_2$ stages of trays (not shown in FIG. 2) being installed in a stripping section and an enrichment section respectively as an internal in a trunk portion of the continuous multi-stage distillation column B.

Description of Reference Numerals

In Fig. 1
1: gas outlet, 2: liquid outlet, 3-a to 3-e: inlet, 4-a and 4-b: inlet, 5: end plate, 6: internal, 7: trunk portion, 10: continuous multi-stage distillation column A, $L_0$: length (cm) of trunk portion, $D_0$: inside diameter (cm) of trunk portion, $d_{01}$: inside diameter (cm) of gas outlet, $d_{02}$: inside diameter (cm) of liquid outlet
In Fig. 2
1: gas outlet, 2: liquid outlet, 3-a to 3-c and 4: inlet, $L_1$: length (cm) of stripping section of the continuous multi-stage distillation column B, $L_2$: length (cm) of enrichment section of the continuous multi-stage distillation column B, $D_1$: inside diameter (cm) of stripping section of the continuous multi-stage distillation column B, $D_2$: inside diameter of enrichment section of the continuous multi-stage distillation column B.

Best Mode for Carrying Out the Invention

Following is a detailed description of the present invention.
The reaction of the present invention is a reversible equilibrium transesterification reaction represented by the following formula in which a dialkyl carbonate (C) and a diol (D) are produced from a cyclic carbonate (A) and an aliphatic monohydric alcohol (B):

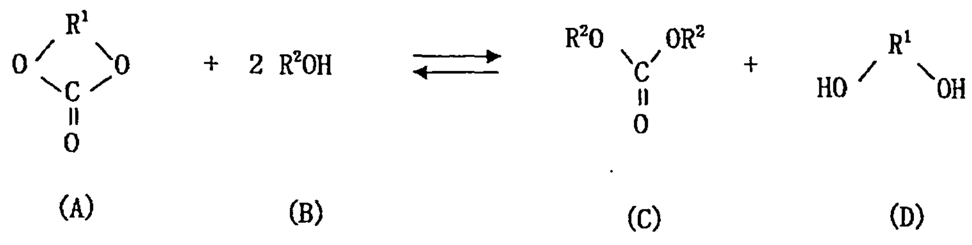

wherein $R^1$ represents a bivalent group $-(CH_2)_m-$ (m is an integer from 2 to 6), one or more of the hydrogens thereof being optionally substituted with an alkyl group or aryl group having 1 to 10 carbon atoms. Moreover, $R^2$ represents a monovalent aliphatic group having 1 to 12 carbon atoms, one or more of the hydrogens thereof being optionally substituted with an alkyl group or aryl group having 1 to 10 carbon atoms.
The cyclic carbonate used as a starting material in the present invention is a compound represented by (A) in

the above formula. For example, an alkylene carbonate such as ethylene carbonate or propylene carbonate, or 1,3-dioxacyclohexa-2-one, 1,3-dioxacyclohepta-2-one, or the like can be preferably used, ethylene carbonate or propylene carbonate being more preferably used due to ease of procurement and so on, and ethylene carbonate being particularly preferably used.

Moreover, the aliphatic monohydric alcohol used as the other starting material is a compound represented by (B) in the above formula. An aliphatic monohydric alcohol having a lower boiling point than that of the diol produced is used. Although possibly varying depending on the type of the cyclic carbonate used, examples include thus methanol, ethanol, propanol (isomers), allyl alcohol, butanol (isomers), 3-buten-1-ol, amyl alcohol (isomers), hexyl alcohol (isomers), heptyl alcohol (isomers), octyl alcohol (isomers), nonyl alcohol (isomers), decyl alcohol (isomers), undecyl alcohol (isomers), dodecyl alcohol (isomers), cyclopentanol, cyclohexanol, cycloheptanol, cyclooctanol, methylcyclopentanol (isomers), ethylcyclopentanol (isomers), methylcyclohexanol (isomers), ethylcyclohexanol (isomers), dimethylcyclohexanol (isomers), diethylcyclohexanol (isomers), phenylcyclohexanol (isomers), benzyl alcohol, phenethyl alcohol (isomers), phenylpropanol (isomers), and so on. Furthermore, these aliphatic monohydric alcohols may be substituted with substituents such as halogens, lower alkoxy groups, cyano groups, alkoxycarbonyl groups, aryloxycarbonyl groups, acyloxy groups, and nitro groups.

Of such aliphatic monohydric alcohols, ones preferably used are alcohols having 1 to 6 carbon atoms, more preferably alcohols having 1 to 4 carbon atoms, i.e. methanol, ethanol, propanol (isomers), and butanol (isomers). In the case of using ethylene carbonate or propylene carbonate as the cyclic carbonate, preferable aliphatic monohydric alcohols are methanol and ethanol, methanol being particularly preferable.

In the process of the present invention, a catalyst is made to be present in the reactive distillation column A. The method of making the catalyst be present may be any method, but in the case, for example, of a homogeneous catalyst that dissolves in the reaction liquid under the reaction conditions, the catalyst can be made to be present in the liquid phase in the reactive distillation column by feeding the catalyst into the reactive distillation column continuously, or in the case of a heterogeneous catalyst that does not dissolve in the reaction liquid under the reaction conditions, the catalyst can be made to be present in the reaction system by disposing the catalyst as a solid in the reactive distillation column; these methods may also be used in combination.

In the case that a homogeneous catalyst is continuously fed into the reactive distillation column, the homogeneous catalyst may be fed in together with the cyclic carbonate and/or the aliphatic monohydric alcohol, or may be fed in at a different position to the starting materials. The reaction actually proceeds in the distillation column in a region below the position at which the catalyst is fed in, and hence it is preferable to feed the catalyst into a region between the top of the column and the position(s) at which the starting materials are fed in. The catalyst must be present in at least 5 stages, preferably at least 7 stages, more preferably at least 10 stages.

Moreover, in the case of using a heterogeneous solid catalyst, the catalyst must be present in at least 5 stages, preferably at least 7 stages, more preferably at least 10 stages. A solid catalyst that also has an effect as a packing in the distillation column may be used.

As the catalyst used in the present invention, any of various catalysts known from hitherto can be used. Examples of the catalyst include;

alkali metals and alkaline earth metals such as lithium, sodium, potassium, rubidium, cesium, magnesium, calcium, strontium, and barium;

basic compounds of alkali metals and alkaline earth metals such as hydrides, hydroxides, alkoxides, aryloxides, and amides;

basic compounds of alkali metals and alkaline earth metals such as carbonates, bicarbonates, and organic acid salts;

tertiary amines such as triethylamine, tributylamine, trihexylamine, and benzyldiethylamine;

nitrogen-containing heteroaromatic compounds such as N-alkylpyrroles, N-alkylindoles, oxazoles, N-alkylimidazoles, N-alkylpyrazoles, oxadiazoles, pyridine, alkylpyridines, quinoline, alkylquinolines, isoquinoline, alkylisoquinolines, acridine, alkylacridines, phenanthroline, alkylphenanthrolines, pyrimidine, alkylpyrimidines, pyrazine, alkylpyrazines, triazines, and alkyltriazines;

cyclic amidines such as diazobicycloundecene (DBU) and diazobicyclononene (DBN);

thallium compounds such as thallium oxide, thallium halides, thallium hydroxide, thallium carbonate, thallium nitrate, thallium sulfate, and thallium organic acid salts;

tin compounds such as tributylmethoxytin, tributylethoxytin, dibutyldimethoxytin, diethyldiethoxytin, dibutyldiethoxytin, dibutylphenoxytin, diphenylmethoxytin, dibutyltin acetate, tributyltin chloride, and tin 2-ethylhexanoate;

zinc compounds such as dimethoxyzinc, diethoxyzinc, ethylenedioxyzinc, and dibutoxyzinc;

aluminum compounds such as aluminum trimethoxide, aluminum triisopropoxide, and aluminum tributoxide;

titanium compounds such as tetramethoxytitanium, tetraethoxytitanium, tetrabutoxytitanium, dichlorodimethoxytitanium, tetraisopropoxytitanium, titanium acetate, and titanium acetylacetonate;

phosphorus compounds such as trimethylphosphine, triethylphosphine, tributylphosphine, triphenylphosphine, tributylmethylphosphonium halides, trioctylbutylphosphonium halides, and triphenylmethylphosphonium halides;
zirconium compounds such as zirconium halides, zirconium acetylacetonate, zirconium alkoxides, and zirconium acetate;
lead and lead-containing compounds, for example lead oxides such as PbO, $PbO_2$, and $Pb_3O_4$;
lead sulfides such as PbS, $Pb_2S_3$, and PbS2;
lead hydroxides such as $Pb(OH)_2$, $Pb_3O_2(OH)_2$, $Pb_2[PbO_2(OH)_2]$, and $Pb_2O(OH)_2$;
plumbites such as $Na_2PbO_2$, $K_2PbO_2$, $NaHPbO_2$, and $KHPbO_2$;
plumbates such as $Na_2PbO_3$, $Na_2H_2PbO$ , $K_2PbO_3$, $K_2[Pb(OH)_6]$, $K_4PbO_4$, $Ca_2PbO_4$, and $CaPbO_3$;
lead carbonates and basic salts thereof such as $PbCO_3$ and $2PbCO_3{\cdot}Pb(OH)_2$;
alkoxylead compounds and aryloxylead compounds such as $Pb(OCH_3)_2$, $(CH_3O)Pb(OPh)$, and $Pb(OPh)_2$;
lead salts of organic acids, and carbonates and basic salts thereof, such as $Pb(OCOCH_3)_2$, $Pb(OCOCH_3)_4$, and $Pb(OCOCH_3)_2.PbO{\cdot}3H_2O$;
organolead compounds such as $Bu_4Pb$, $Ph_4Pb$, $Bu_3PbCl$, $Ph_3PbBr$, $Ph_3Pb$ (or $Ph_6Pb_2$), $Bu_3PbOH$, and $Ph_2PbO$ (wherein Bu represents a butyl group, and Ph represents a phenyl group);
lead alloys such as Pb-Na, Pb-Ca, Pb-Ba, Pb-Sn, and Pb-Sb; lead minerals such as galena and zinc blende; and hydrates of such lead compounds.

In the case that the compound used dissolves in a starting material of the reaction, the reaction mixture, a reaction by-product or the like, the compound can be used as a homogeneous catalyst, whereas in the case that the compound does not dissolve, the compound can be used as a solid catalyst. Furthermore, it is also preferable to use, as a homogeneous catalyst, a mixture obtained by dissolving a compound as above in a starting material of the reaction, the reaction mixture, a reaction by-product or the like, or by reacting to bring about dissolution.

Furthermore, ion exchangers such as anion exchange resins having tertiary amino groups, ion exchange resins having amide groups, ion exchange resins having at least one type of exchange groups selected from sulfonate groups, carboxylate groups and phosphate groups, and solid strongly basic anion exchangers having quaternary ammonium groups as exchange groups; solid inorganic compounds such as silica, silica-alumina, silica-magnesia, aluminosilicates, gallium silicate, various zeolites, various metal-exchanged zeolites, and ammonium-exchanged zeolites, and so on can also be used as the catalyst.

As a solid catalyst, a particularly preferably used one is a solid strongly basic anion exchanger having quaternary ammonium groups as exchange groups, examples thereof including a strongly basic anion exchange resin having quaternary ammonium groups as exchange groups, a cellulose strongly basic anion exchanger having quaternary ammonium groups as exchange groups, and an inorganic carrier supported type strongly basic anion exchanger having quaternary ammonium groups as exchange groups. As a strongly basic anion exchange resin having quaternary ammonium groups as exchange groups, for example a styrene type strongly basic anion exchange resin or the like can be preferably used. A styrene type strongly basic anion exchange resin is a strongly basic anion exchange resin having a copolymer of styrene and divinylbenzene as a parent material, and having quaternary ammonium groups (type I or type II) as exchange groups, and can be schematically represented, for example, by the following formula:

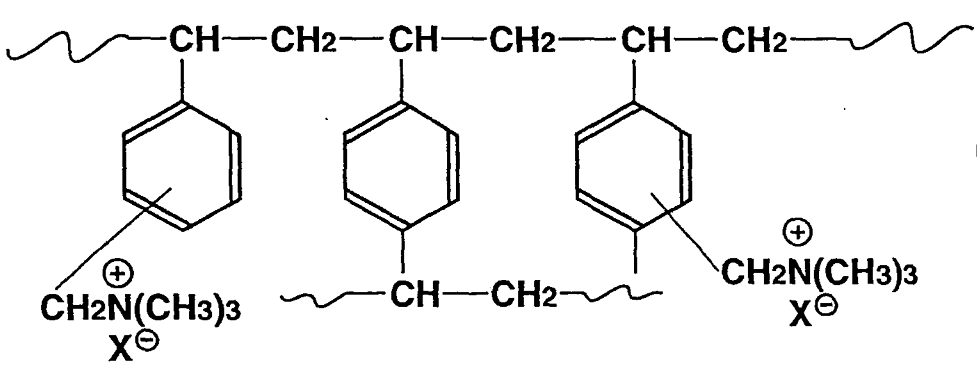

(type I)

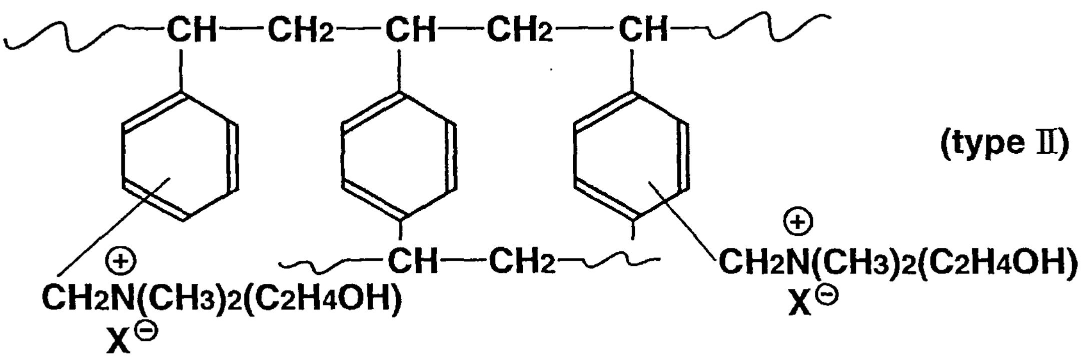

(type II)

wherein X represents an anion; as X, generally at least one type of anion selected from $F^-$, $Cl^-$, $Br^-$, $I^-$, $HCO_3^-$, $CO_3^{2-}$, $CH_3CO_2^-$, $HCO_2^-$, $IO_3^-$, $BrO_3^-$, and $ClO_3^-$ is used, preferably at least one type of anion selected from $Cl^-$, $Br^-$, $HCO_3^-$, and $CO_3^{2-}$. Moreover, as the structure of the resin parent material, either a gel type one or a macroreticular (MR) type one can be used, the MR type being particularly preferable due to the organic solvent resistance being high.

An example of a cellulose strongly basic anion exchanger having quaternary ammonium groups as exchange groups is cellulose having $-OCH_2CH_2NR_3X$ exchange groups obtained by converting some or all of the -OH groups in the cellulose into trialkylaminoethyl groups. Here, R represents an alkyl group; methyl, ethyl, propyl, butyl or the like is generally used, preferably methyl or ethyl. Moreover, X represents an anion as defined above.

An inorganic carrier supported type strongly basic anion exchanger having quaternary ammonium groups as exchange groups that can be used in the present invention means an inorganic carrier that has had $-O(CH_2)_nNR_3X$ quaternary ammonium groups introduced thereto by modifying some or all of the -OH surface hydroxyl groups of the inorganic carrier. Here, R and X are defined as above n is generally an integer from 1 to 6, preferably n = 2. As the inorganic carrier, silica, alumina, silica-alumina, titania, a zeolite, or the like can be used, it being preferable to use silica, alumina, or silica-alumina, particularly preferably silica. Any method can be used as the method of modifying the surface hydroxyl groups of the inorganic carrier.

As the solid strongly basic anion exchanger having quaternary ammonium groups as exchange groups, a commercially available one may be used. In this case, the anion exchanger may also be used as the transesterification catalyst after being subjected to ion exchange with a desired anionic species in advance as pretreatment.

Moreover, a solid catalyst containing a macroreticular or gel-type organic polymer having bonded thereto heterocyclic groups each containing at least one nitrogen atom, or an inorganic carrier having bonded thereto heterocyclic groups each containing at least one nitrogen atom can also be preferably used as the transesterification catalyst. Furthermore, a solid catalyst in which some or all of these nitrogen-containing heterocyclic groups have been converted into a quaternary salt can be similarly used. Note that a solid catalyst such as an ion exchanger may also act as a packing in the present invention.

An amount of the catalyst used in the present invention varies depending on the type of the catalyst used, but in the case of continuously feeding in a homogeneous catalyst that dissolves in the reaction liquid under the reaction conditions, the amount used is generally in a range of from 0.0001 to 50 % by weight, preferably from 0.005 to 20 % by weight, more preferably from 0.01 to 10 % by weight, as a proportion of the total weight of the cyclic carbonate and the aliphatic monohydric alcohol fed in as the starting materials. Moreover, in the case of using a solid catalyst installed in the distillation column, the catalyst is preferably used in an amount in a range of from 0.01 to 75 vol%, more preferably from 0.05 to 60 vol%, yet more preferably from 0.1 to 60 vol%, based on the empty column volume of the distillation column.

When continuously feeding the cyclic carbonate into a continuous multi-stage distillation column constituting the reactive distillation column A in the present invention, it is preferable for the cyclic carbonate to be fed into a specified stage. For example, it is preferable for the starting material cyclic carbonate to be continuously introduced into the continuous multi-stage distillation column from at least one inlet provided between the 3$^{rd}$ stage from the top of the continuous multi-stage distillation column and the (n/3)$^{th}$ stage from the top of the continuous multi-stage distillation column. This is important to ensure that high boiling point compounds such as the cyclic carbonate and the diol are not contained in a column top component in stages above the cyclic carbonate inlet(s). For this reason, there are preferably not less than 3 stages, more preferably 4 to 10 stages, yet more preferably 5 to 8 stages, above the cyclic carbonate inlet(s).

A cyclic carbonate preferably used in the present invention is one not containing a halogen that has been produced through reaction between, for example, an alkylene oxide such as ethylene oxide, propylene oxide or styrene oxide and carbon dioxide. A cyclic carbonate containing small amounts of such raw material compounds, the diol, and so on may thus be used as a starting material in the present invention.

In the present invention, the starting materials fed in may contain the product dialkyl carbonate and / or diol.

The content thereof is, for the dialkyl carbonate, generally in a range of from 0 to 40 % by weight, preferably from 0 to 30 % by weight, more preferably from 0 to 20 % by weight, in terms of the percentage by weight of the dialkyl carbonate in the aliphatic monohydric alcohol / dialkyl carbonate mixture, and is, for the diol, generally in a range of from 0 to 10 % by weight, preferably from 0 to 7 % by weight, more preferably from 0 to 5 % by weight, in terms of the percentage by weight of the diol in the cyclic carbonate / diol mixture.

When carrying out the present reaction industrially, besides fresh cyclic carbonate and / or aliphatic monohydric alcohol newly introduced into the reaction system, material having the cyclic carbonate and / or the aliphatic monohydric alcohol as a main component thereof recovered from step (II) of the present invention and / or another process can also be preferably used for the starting materials. For example, a column top component $B_T$ obtained through separation / purification in step (II) is generally a mixture of the aliphatic monohydric alcohol as a main component thereof together with the dialkyl carbonate, and hence it is preferable to reuse this as some of a starting material in step (I); it is an excellent characteristic feature of the present invention that this is possible. An example of another process is a process in which a diaryl carbonate is produced from the dialkyl carbonate and an aromatic monohydroxy compound, the aliphatic monohydric alcohol being by-produced in this process and recovered. The recovered by-produced aliphatic monohydric alcohol generally contains the dialkyl carbonate, but it has been discovered that in the case that the content of the dialkyl carbonate is in a range as above, excellent effects of the present invention can be achieved. The recovered by-produced aliphatic monohydric alcohol may further contain the aromatic monohydroxy compound, an alkyl aryl ether, small amounts of an alkyl aryl carbonate and the diaryl carbonate, and so on. The by-produced aliphatic monohydric alcohol may be used as is as a starting material in the present invention, or may be used as the starting material after the amount of contained matter having a higher boiling point than the aliphatic monohydric alcohol has been reduced through distillation or the like.

When continuously feeding the aliphatic monohydric alcohol into the continuous multi-stage distillation column A constituting the reactive distillation column in the present invention, it is preferable for the aliphatic monohydric alcohol to be fed into a specified stage. For example, it is preferable for the starting material aliphatic monohydric alcohol to be continuously introduced into the continuous multi-stage distillation column A from at least one inlet provided between the $(n/3)^{th}$ stage from the top of the continuous multi-stage distillation column A and the $(2n/3)^{th}$ stage from the top of the continuous multi-stage distillation column A. It has been discovered that in the case that the aliphatic monohydric alcohol used as a starting material in the present invention contains a specified amount of the dialkyl carbonate as described above, excellent effects of the present invention can be achieved by making the aliphatic monohydric alcohol inlet(s) be in a stage within this specified range. More preferably, the aliphatic monohydric alcohol is continuously introduced into the continuous multi-stage distillation column A from at least one inlet provided between the $(2n/5)^{th}$ stage from the top of the continuous multi-stage distillation column A and the $(3n/5)^{th}$ stage from the top of the continuous multi-stage distillation column A.

The starting materials are fed continuously into the distillation column in a liquid form, in a gaseous form, or as a mixture of a liquid and a gas. Other than feeding the starting materials into the distillation column in this way, it is also preferable to additionally feed in a gaseous starting material intermittently or continuously from a central portion and / or a lower portion of the distillation column. Moreover, another preferable method is one in which the cyclic carbonate is continuously fed in a liquid form or a gas / liquid mixed form into a stage of the distillation column above the stages in which the catalyst is present, and the aliphatic monohydric alcohol is continuously fed into the distillation column in a gaseous form and / or a liquid form from at least one inlet provided in a stage of the distillation column as described above. The starting materials are preferably made to contact the catalyst in a region of at least 5 stages, preferably at least 7 stages, more preferably at least 10 stages, of the distillation column.

In the present invention, a ratio between the amounts of the cyclic carbonate and the aliphatic monohydric alcohol fed into the reactive distillation column varies according to the type and amount of the transesterification catalyst and the reaction conditions, but a molar ratio of the aliphatic monohydric alcohol to the cyclic carbonate fed in is generally in a range of from 0.01 to 1000 times. To increase the cyclic carbonate conversion, it is preferable to feed in the aliphatic monohydric alcohol in an excess of at least 2 times the number of mols of the cyclic carbonate, but if the amount of the aliphatic monohydric alcohol used is too great, then it is necessary to make the apparatus larger. For such reasons, the molar ratio of the aliphatic monohydric alcohol to the cyclic carbonate is preferably in a range of from 2 to 20, more preferably from 3 to 15, yet more preferably from 5 to 12. Furthermore, if much unreacted cyclic carbonate remains, then the unreacted cyclic carbonate may react with the product diol to by-produce oligomers such as a dimer or a trimer, and hence in industrial implementation, it is preferable to reduce the amount of unreacted cyclic carbonate remaining as much as possible. In the process of the present invention, even if the above molar ratio is not more than 10, the cyclic carbonate conversion can be made to be not less than 98%, preferably not less than 99%, more preferably not less than 99.9%. This is another characteristic feature of the present invention.

In the present invention, preferably not less than 2 ton / hr of the dialkyl carbonate is continuously produced (step (I)) and separated out and purified (step (II)); the minimum amount of the cyclic carbonate continuously fed in to achieve this is generally 2.2 P ton / hr, preferably 2.1 P ton / hr, more preferably 2.0 P ton / hr, based on the amount P

(ton / hr) of the dialkyl carbonate to be produced. In a yet more preferable case, this amount can be made to be less than 1.9 P ton / hr.

The continuous multi-stage distillation column A used in step (I) in the present invention satisfies not only conditions from the perspective of the distillation function, but also these conditions are combined with conditions required so as make the reaction proceed stably with a high conversion and high selectivity. Specifically, the continuous multi-stage distillation column A must be a tray column having a cylindrical trunk portion having a length $L_0$ (cm) and an inside diameter $D_0$ (cm) and having thereinside a number of stages $n_0$ of a tray having a plurality of holes therein, and further having a gas outlet having an inside diameter $d_{01}$ (cm) at a top of the column or in an upper portion of the column near to the top, a liquid outlet having an inside diameter $d_{02}$ (cm) at a bottom of the column or in a lower portion of the column near to the bottom, at least one first inlet provided in the upper portion and / or the middle portion of the column below the gas outlet, and at least one second inlet provided in the middle portion and / or the lower portion of the column above the liquid outlet, wherein $L_0$, $D_0$, $n_0$, $d_{01}$ and $d_{02}$ satisfy the following formulae (1) to (6);

$$2100 \leq L_0 \leq 8000 \quad (1)$$

$$180 \leq D_0 \leq 2000 \quad (2)$$

$$4 \leq L_0 / D_0 \leq 40 \quad (3)$$

$$20 \leq n_0 \leq 120 \quad (4)$$

$$3 \leq D_0 / d_{01} \leq 20 \quad (5);$$

and

$$5 \leq D_0 / d_{02} \leq 30 \quad (6).$$

Note that the term "the top of the column or the upper portion of the column near to the top" used in the present invention means the portion from the top of the column downward as far as approximately 0.25 $L_0$, and the term "the bottom of the column or the lower portion of the column near to the bottom" means the portion from the bottom of the column upward as far as approximately 0.25 $L_0$. Here, "$L_0$" is defined as above.

It has been discovered that by using the continuous multi-stage distillation column A that simultaneously satisfies the formulae (1), (2), (3), (4), (5) and (6), the dialkyl carbonate and the diol can be produced on an industrial scale of preferably not less than 2 ton / hr of the dialkyl carbonate and / or preferably not less than 1.3 of the diol with a high conversion, high selectivity, and high productivity stably for a prolonged period of time of, for example, not less than 1000 hours, preferably not less than 3000 hours, more preferably not less than 5000 hours, from the cyclic carbonate and the aliphatic monohydric alcohol. The reason why it has become possible to produce the dialkyl carbonate and the diol on an industrial scale with such excellent effects by implementing the process according to the present invention is not clear, but this is supposed to be due to a composite effect brought about when the above conditions are combined. Preferable ranges for the respective factors are described below.

If $L_0$ (cm) is less than 2100, then the conversion decreases and hence it is not possible to attain the desired production amount. Moreover, to keep down the equipment cost while securing the conversion enabling the desired production amount to be attained, $L_0$ must be made to be not more than 8000. A more preferable range for $L_0$ (cm) is $2300 \leq L_0 \leq 6000$, with $2500 \leq L_0 \leq 5000$ being yet more preferable.

If $D_0$ (cm) is less than 180, then it is not possible to attain the desired production amount. Moreover, to keep

down the equipment cost while attaining the desired production amount, $D_0$ must be made to be not more than 2000. A more preferable range for $D_0$ (cm) is $200 \leq D_0 \leq 1000$, with $210 \leq D_0 \leq 800$ being yet more preferable.

If $L_0 / D_0$ is less than 4 or greater than 40, then stable operation becomes difficult. In particular, if $L_0 / D_0$ is greater than 40, then the pressure difference between the top and bottom of the column becomes too great, and hence prolonged stable operation becomes difficult. Moreover, it becomes necessary to increase the temperature in the lower portion of the column, and hence side reactions become liable to occur, bringing about a decrease in the selectivity. A more preferable range for $L_0 / D_0$ is $5 \leq L_0 / D_0 \leq 30$, with $7 \leq L_0 / D_0 \leq 20$ being yet more preferable.

If $n_0$ is less than 10, then the conversion decreases and hence it is not possible to attain the desired production amount. Moreover, to keep down the equipment cost while securing the conversion enabling the desired production amount to be attained, $n_0$ must be made to be not more than 120. Furthermore, if $n_0$ is greater than 120, then the pressure difference between the top and bottom of the column becomes too great, and hence prolonged stable operation becomes difficult. Moreover, it becomes necessary to increase the temperature in the lower portion of the column, and hence side reactions become liable to occur, bringing about a decrease in the selectivity. A more preferable range for $n_0$ is $30 \leq n_0 \leq 100$, with $40 \leq n_0 \leq 90$ being yet more preferable.

If $D_0 / d_{01}$ is less than 3, then the equipment cost becomes high. Moreover, a large amount of a gaseous component is readily released to the outside of the system, and hence stable operation becomes difficult. If $D_0 / d_{01}$ is greater than 20, then the gaseous component withdrawal amount becomes relatively low, and hence stable operation becomes difficult, and moreover a decrease in the conversion is brought about. A more preferable range for $D_0 / d_{01}$ is $4 \leq D_0 / d_{01} \leq 15$, with $5 \leq D_0 / d_{01} \leq 13$ being yet more preferable.

If $D_0 / d_{02}$ is less than 5, then the equipment cost becomes high. Moreover, the liquid withdrawal amount becomes relatively high, and hence stable operation becomes difficult. If $D_0 / d_{02}$ is greater than 30, then the flow rate through the liquid outlet and piping becomes excessively fast, and hence erosion becomes liable to occur, bringing about corrosion of the apparatus. A more preferable range for $D_0 / d_{02}$ is $7 \leq D_0 / d_{02} \leq 25$, with $9 \leq D_0 / d_{02} \leq 20$ being yet more preferable.

Furthermore, it has been found that in the present invention it is further preferable for the $d_{01}$ and the $d_{02}$ to satisfy the following formula (7);

$$1 \leq d_{01} / d_{02} \leq 5 \qquad (7).$$

The term "prolonged stable operation" used in the present invention means that the continuous multi-stage distillation column can be operated continuously in a steady state based on the operating conditions with no flooding or weeping, clogging of piping, or erosion for not less than 1000 hours, preferably not less than 3000 hours, more preferably not less than 5000 hours, and predetermined amounts of the dialkyl carbonate and the diol can be produced while maintaining a high conversion, high selectivity, and high productivity.

A characteristic feature of step (I) of the present invention is that the dialkyl carbonate and the diol can be produced stably for a prolonged period of time each with high selectivity and preferably with high productivity for the dialkyl carbonate of not less than 2 ton / hr and high productivity for the diol of not less than 1.3 ton / hr. The dialkyl carbonate and the diol are more preferably produced in an amount of not less than 3 ton / hr and not less than 1.95 ton / hr respectively, yet more preferably not less than 4 ton / hr and not less than 2.6 ton / hr respectively. Moreover, another characteristic feature of step (I) of the present invention is that in the case that $L_0$, $D_0$, $L_0 / D_0$, $n_0$, $D_0 / d_{01}$, and $D_0 / d_{02}$ for the continuous multi-stage distillation column satisfy respectively $2300 \leq L_0 \leq 6000$, $200 \leq D_0 \leq 1000$, $5 \leq L_0 / D_0 \leq 30$, $30 \leq n_0 \leq 100$, $4 \leq D_0 / d_{01} \leq 15$, and $7 \leq D_0 / d_{02} \leq 25$, not less than 2.5 ton / hr, preferably not less than 3 ton / hr, more preferably not less than 3.5 ton / hr of the dialkyl carbonate, and not less than 1.6 ton / hr, preferably not less than 1.95 ton / hr, more preferably not less than 2.2 ton / hr of the diol can be produced. Furthermore, another characteristic feature of step (I) of the present invention is that in the case that $L_0$, $D_0$, $L_0 / D_0$, $n_0$, $D_0 / d_{01}$, and $D_0 / d_{02}$ for the continuous multi-stage distillation column satisfy respectively $2500 \leq L_0 \leq 5000$, $210 \leq D_0 \leq 800$, $7 \leq L_0 / D_0 \leq 20$, $40 \leq n_0 \leq 90$, $5 \leq D_0 / d_{01} \leq 13$, and $9 \leq D_0 / d_{02} \leq 20$, not less than 3 ton / hr, preferably not less than 3.5 ton / hr, more preferably not less than 4 ton / hr of the dialkyl carbonate, and not less than 1.95 ton / hr, preferably not less than 2.2 ton / hr, more preferably not less than 2.6 ton / hr of the diol can be produced.

The "selectivity" for each of the dialkyl carbonate and the diol in the present invention is based on the cyclic carbonate reacted. In the present invention, a high selectivity of not less than 95% can generally be attained, preferably not less than 97%, more preferably not less than 99%. Moreover, the "conversion" in the present invention generally indicates the cyclic carbonate conversion, in the present invention it being possible to make the cyclic carbonate conversion be not less than 95%, preferably not less than 97%, more preferably not less than 99%, yet more preferably not less than 99.5%, still more preferably not less than 99.9%. It is one of the excellent characteristic features of the present invention that a high conversion can be maintained while maintaining high selectivity in this way.

The continuous multi-stage distillation column A used in step (I) is a tray column type distillation column having trays as the internal. The term "internal" used in the present invention means the part in the distillation column where gas and liquid are actually brought into contact with one another. Examples of the tray include a bubble-cap tray, a sieve tray, a ripple tray, a ballast tray, a valve tray, a counterflow tray, an Unifrax tray, a Superfrac tray, a Maxfrac tray, a dual flow tray, a grid plate tray, a turbogrid plate tray, a Kittel tray, or the like. Moreover, in step (I), a multi-stage distillation column having both a tray portion and a portion packed with packings in part of the tray stage portion can also be used. Examples of the packings include random packings such as a Raschig ring, a Lessing ring, a Pall ring, a Berl saddle, an Intalox saddle, a Dixon packing, a McMahon packing or Heli-Pak, or structured packings such as Mellapak, Gempak, Techno-pack, Flexipac, a Sulzer packing, a Goodroll packing or Glitschgrid. Furthermore, the term "number of stages n (no, $n_1$, $n_2$ etc.) of the internal" used in the present invention means the number of trays in the case of trays, and the theoretical number of stages in the case of packings. The number of stages n in the case of a multi-stage distillation column having both a tray portion and a portion packed with packings is thus the sum of the number of trays and the theoretical number of stages.

For the reaction between the cyclic carbonate and the aliphatic monohydric alcohol in step (I), it has been discovered that the high conversion, high selectivity, and high productivity can be attained even if a plate type continuous multi-stage distillation column in which the internal comprises trays and / or packings having a predetermined number of stages and / or a packed column type continuous multi-stage distillation column is used, but a plate type distillation column in which the internal is the tray is preferable. Furthermore, it has been discovered that sieve trays each having a sieve portion and a downcomer portion are particularly good as the tray in terms of the relationship between performance and equipment cost. It has also been discovered that each sieve tray preferably has 100 to 1000 holes / $m^2$ in the sieve portion. A more preferable number of holes is from 120 to 900 holes / $m^2$, yet more preferably from 150 to 800 holes / $m^2$. Moreover, it has been discovered that the cross-sectional area per hole of each sieve tray is preferably in a range of from 0.5 to 5 $cm^2$. A more preferable cross-sectional area per hole is from 0.7 to 4 $cm^2$, yet more preferably from 0.9 to 3 $cm^2$. Furthermore, it has been discovered that it is particularly preferable if each sieve tray has 100 to 1000 holes / $m^2$ in the sieve portion, and the cross-sectional area per hole is in a range of from 0.5 to 5 $cm^2$. The number of holes in the sieve portion may be the same for all of the sieve trays, or may differ.

The term "aperture ratio" used in the present invention means, for each of the sieve trays in the continuous multi-stage distillation column A, the ratio of the total area of openings in the tray through which gas and liquid can pass (the total cross-sectional area of holes) to the area of the tray having these openings therein. Note that for a tray having a downcomer portion, the area of the portion in which bubbling substantially occurs, i.e. excluding the downcomer portion, is taken as the area of the tray.

It has been found that the aperture ratio of each of the sieve trays in the continuous multi-stage distillation column A is preferably in a range of from 1.5 to 15%. If the aperture ratio is less than 1.5%, then the apparatus becomes large relative to the required production amount, and hence the equipment cost becomes high. Moreover, the residence time increases, and hence side reactions (e.g. a reaction between the reaction product diol and unreacted cyclic carbonate) become liable to occur. Moreover, if the aperture ratio is greater than 15%, then the residence time in each of the trays decreases, and hence the number of stages must be increased to attain a high conversion, and thus the problems described above for when $n_0$ is large arise. For such reasons, a more preferable range for the aperture ratio is 1.7 to 8.0%, with 1.9 to 6.0% being yet more preferable.

The aperture ratio may be the same for all of the trays in the continuous multi-stage distillation column A, or may differ. In the present invention, it is generally preferable to use a multi-stage distillation column in which the aperture ratio of trays in the upper portion thereof is greater than the aperture ratio of trays in the lower portion thereof.

In step (I), the dialkyl carbonate and the diol are continuously produced by continuously feeding the starting material cyclic carbonate and aliphatic monohydric alcohol into the continuous multi-stage distillation column A in which a catalyst is present, carrying out reaction and distillation simultaneously in the column, continuously withdrawing a low boiling point reaction mixture $A_T$ containing the produced dialkyl carbonate from an upper portion of the column in a gaseous form, and continuously withdrawing a high boiling point reaction mixture $A_B$ containing the diol from a lower portion of the column A in a liquid form.

The reaction time for the transesterification reaction carried out in step (I) is considered to equate to the average residence time of the reaction liquid in the continuous multi-stage distillation column A. The reaction time varies depending on the form of the internals in the distillation column A and the number of stages, the amounts of the starting materials fed in, the type and amount of the catalyst, the reaction conditions, and so on. The reaction time is generally in a range of from 0.1 to 20 hours, preferably from 0.5 to 15 hours, more preferably from 1 to 10 hours.

The reaction temperature varies depending on the type of the starting material compounds used, and the type and amount of the catalyst. The reaction temperature is generally in a range of from 30 to 300 °C. It is preferable to increase the reaction temperature so as to increase the reaction rate. However, if the reaction temperature is too high, then side reactions become liable to occur. The reaction temperature is thus preferably in a range of from 40 to 250 °C, more preferably from 50 to 200 °C, yet more preferably from 60 to 150 °C. In the present invention, the reactive distillation

can be carried out with the column bottom temperature set to not more than 150 °C, preferably not more than 130 °C, more preferably not more than 110 °C, yet more preferably not more than 100 °C. An excellent characteristic feature of the present invention is that the high conversion, high selectivity, and high productivity can be attained even with such a low column bottom temperature. Moreover, the reaction pressure varies depending on the type of the starting material compounds used and the composition therebetween, the reaction temperature, and so on. The reaction pressure may be any of a reduced pressure, normal pressure, or an applied pressure, and is generally in a range of from 1 to $2\times10^7$ Pa, preferably from $10^3$ to $10^7$ Pa, more preferably from $10^4$ to $5\times10^6$ Pa.

The material constituting the continuous multi-stage distillation column A used in the present invention is generally a metallic material such as carbon steel or stainless steel. In terms of the quality of the dialkyl carbonate and the diol to be produced, stainless steel is preferable.

In the present invention, step (II) is carried out following on from step (I), whereby a high-purity dialkyl carbonate of purity not less than 97%, preferably not less than 99%, more preferably not less than 99.9% is separated out with high efficiency.

In step (II), a continuous multi-stage distillation column B is used to subject the low boiling point reaction mixture $A_T$ containing the produced dialkyl carbonate and the aliphatic monohydric alcohol continuously withdrawn from the upper portion of the continuous multi-stage distillation column A in a gaseous form to separation by distillation into a column top component $B_T$ having the aliphatic monohydric alcohol as a main component thereof and a column bottom component $B_B$ having the dialkyl carbonate as a main component thereof.

The continuous multi-stage distillation column B used in step (II) must have a function of separating out the dialkyl carbonate with a prescribed separation efficiency stably for a prolonged period of time from a large amount of the low boiling point reaction mixture $A_T$, and various conditions must be simultaneously satisfied to achieve this.

Specifically, the continuous multi-stage distillation column B is a distillation column comprising a stripping section having a length $L_1$ (cm), an inside diameter $D_1$ (cm) and internals with a number of stages $n_1$ thereinside, and an enrichment section having a length $L_2$ (cm), an inside diameter $D_2$ (cm) and internals with a number of stages $n_2$ thereinside, wherein $L_1$, $D_1$, $n_1$, $L_2$, $D_2$, and $n_2$ satisfy the following formulae (7) to (14);

$$500 \leq L_1 \leq 3000 \qquad (7)$$

$$100 \leq D_1 \leq 1000 \qquad (8)$$

$$2 \leq L_1 / D_1 \leq 30 \qquad (9)$$

$$10 \leq n_1 \leq 40 \qquad (10)$$

$$700 \leq L_2 \leq 5000 \qquad (11)$$

$$50 \leq D_2 \leq 800 \qquad (12)$$

$$10 \leq L_2 / D_2 \leq 50 \qquad (13);$$

and

$$35 \le n_2 \le 100 \qquad (14).$$

It has been discovered that by using such a continuous multi-stage distillation column B simultaneously satisfying the formulae (7) to (14), a large amount of the low boiling point reaction mixture $A_T$ produced through the reactive distillation between the cyclic carbonate and the aliphatic monohydric alcohol can be separated with high efficiency, so that a column bottom component $B_B$ containing a high concentration of the dialkyl carbonate can be obtained on an industrial scale of preferably not less than 2 ton / hr stably for a prolonged period of time of, for example, not less than 1000 hours, preferably not less than 3000 hours, more preferably not less than 5000 hours. Moreover, regarding the purity of the dialkyl carbonate separated out as the column bottom component $B_B$, generally a high purity of not less than 97 % by weight, preferably not less than 99 % by weight, can be easily attained. In step (II), it is also easy to make the purity of the dialkyl carbonate obtained as the column bottom component be an ultra-high purity of preferably not less than 99.9 % by weight, more preferably not less than 99.99 % by weight. The reason why it has become possible to produce, and separate out and purify, the dialkyl carbonate on an industrial scale with such excellent effects by implementing the process according to the present invention is not clear, but this is supposed to be due to a composite effect brought about when the conditions of the formulae (1) to (14) are combined. Preferable ranges for the respective factors are described below.

If $L_1$ (cm) is less than 500, then the separation efficiency for the stripping section decreases, and hence the desired separation efficiency cannot be attained. Moreover, to keep down the equipment cost while securing the desired separation efficiency, $L_1$ must be made to be not more than 3000. A more preferable range for $L_1$ (cm) is $800 \le L_1 \le 2500$, with $1000 \le L_1 \le 2000$ being yet more preferable.

If $D_1$ (cm) is less than 100, then it is not possible to attain the desired distillation amount. Moreover, to keep down the equipment cost while attaining the desired distillation amount, $D_1$ must be made to be not more than 1000. A more preferable range for $D_1$ (cm) is $120 \le D_1 \le 800$, with $150 \le D_1 \le 600$ being yet more preferable.

If $L_1 / D_1$ is less than 2 or greater than 30, then prolonged stable operation becomes difficult. A more preferable range for $L_1 / D_1$ is $5 \le L_1 / D_1 \le 20$, with $7 \le L_1 / D_1 \le 15$ being yet more preferable.

If $n_1$ is less than 10, then the separation efficiency for the stripping section decreases and hence the desired separation efficiency cannot be attained. Moreover, to keep down the equipment cost while securing the desired separation efficiency, $n_1$ must be made to be not more than 40. A more preferable range for $n_1$ is $13 \le n_1 \le 25$, with $15 \le n_1 \le 20$ being yet more preferable.

If $L_2$ (cm) is less than 700, then the separation efficiency for the enrichment section decreases, and hence the desired separation efficiency cannot be attained. Moreover, to keep down the equipment cost while securing the desired separation efficiency, $L_2$ must be made to be not more than 5000. Furthermore, if $L_2$ is greater than 5000, then the pressure difference between the top and bottom of the column becomes too great, and hence prolonged stable operation becomes difficult. Moreover, it becomes necessary to increase the temperature in the lower portion of the column, and hence side reactions become liable to occur. A more preferable range for $L_2$ (cm) is $1500 \le L_2 \le 3500$, with $2000 \le L_2 \le 3000$ being yet more preferable.

If $D_2$ (cm) is less than 50, then it is not possible to attain the desired distillation amount. Moreover, to keep down the equipment cost while attaining the desired distillation amount, $D_2$ must be made to be not more than 800. A more preferable range for $D_2$ (cm) is $70 \le D_2 \le 600$, with $80 \le D_2 \le 400$ being yet more preferable.

If $L_2 / D_2$ is less than 10 or greater than 50, then prolonged stable operation becomes difficult. A more preferable range for $L_2 / D_2$ is $15 \le L_2 / D_2 \le 30$, with $20 \le L_2 / D_2 \le 28$ being yet more preferable.

If $n_2$ is less than 35, then the separation efficiency for the enrichment section decreases and hence the desired separation efficiency cannot be attained. Moreover, to keep down the equipment cost while securing the desired separation efficiency, $n_2$ must be made to be not more than 100. Furthermore, if $n_2$ is greater than 100, then the pressure difference between the top and bottom of the column becomes too great, and hence prolonged stable operation becomes difficult. Moreover, it becomes necessary to increase the temperature in the lower portion of the column, and hence side reactions become liable to occur. A more preferable range for $n_2$ is $40 \le n_2 \le 70$, with $45 \le n_2 \le 65$ being yet more preferable.

Moreover, for the continuous multi-stage distillation column B used in step (II), it is preferable to be $L_1 \le L_2$, more preferably $L_1 < L_2$. Furthermore, it is preferable to be $D_2 \le D_1$, more preferably $D_2 < D_1$. In the present invention, the case that $L_1 \le L_2$ and $D_2 \le D_1$ is thus preferable, the case that $L_1 < L_2$ and $D_2 < D_1$ being more preferable.

The continuous multi-stage distillation column B used in step (II) is preferably a distillation column having trays and/or packings as the internal in each of the striping section and the enrichment section. A multi-stage distillation column having both a tray portion and a portion packed with packings can also be used.

It is particularly preferable for the internal in both the striping section and the enrichment section of the continuous multi-stage distillation column B used in step (II) to be a tray. Furthermore, it has been discovered that sieve trays each having a sieve portion and a downcomer portion are particularly good as the tray in terms of the relationship between

performance and equipment cost. It has also been discovered that each sieve tray preferably has 150 to 1200 holes / $m^2$ in the sieve portion. A more preferable number of holes is from 200 to 1100 holes / $m^2$, yet more preferably from 250 to 1000 holes / $m^2$. Moreover, it has been discovered that the cross-sectional area per hole of each sieve tray is preferably in a range of from 0.5 to 5 $cm^2$ A more preferable cross-sectional area per hole is from 0.7 to 4 $cm^2$, yet more preferably from 0.9 to 3 $cm^2$. Furthermore, it has been discovered that it is particularly preferable if each sieve tray has 150 to 1200 holes / $m^2$ in the sieve portion, and the cross-sectional area per hole is in a range of from 0.5 to 5 $cm^2$. It has been shown that by adding the above conditions to the continuous multi-stage distillation column B, the object of the present invention can be attained more easily.

In the present invention, the dialkyl carbonate produced through the reactive distillation in the continuous multi-stage distillation column A is continuously withdrawn from the upper portion of the column in a gaseous form as the low boiling point reaction mixture $A_T$ together with aliphatic monohydric alcohol that has remained unreacted due to generally being used in excess. The low boiling point reaction mixture $A_T$ is continuously fed into the continuous multi-stage distillation column B, a low boiling point mixture $B_T$ having the aliphatic monohydric alcohol as a main component thereof is continuously withdrawn from an upper portion of the column in a gaseous form, and a high boiling point mixture $B_B$ having the dialkyl carbonate as a main component thereof is continuously withdrawn from a lower portion of the column in a liquid form. When feeding the low boiling point reaction mixture $A_T$ into the continuous multi-stage distillation column B, the low boiling point reaction mixture $A_T$ may be fed in in a gaseous form, or in a liquid form. It is preferable to heat or cool the low boiling point reaction mixture $A_T$ to a temperature close to the liquid temperature in the vicinity of the feeding inlet of the continuous multi-stage distillation column B before feeding the low boiling point reaction mixture $A_T$ into the distillation column B.

Moreover, the position from which the low boiling point reaction mixture $A_T$ is fed into the continuous multi-stage distillation column B is preferably around between the stripping section and the enrichment section. The continuous multi-stage distillation column B preferably has a reboiler for heating the distillate, and a refluxing apparatus.

In the present invention, the low boiling point reaction mixture $A_T$ is generally withdrawn from the continuous multi-stage distillation column A in an amount of not less than 2 ton / hr, before being fed into the continuous multi-stage distillation column B and thus subjected to the separation by distillation, whereupon the low boiling point mixture $B_T$ is continuously withdrawn from the upper portion of the distillation column B, and the high boiling point mixture $B_B$ is continuously withdrawn from the lower portion of the distillation column B.

In step (II), the concentration of the aliphatic monohydric alcohol in the low boiling point mixture $B_T$ can be made to be not less than 80 % by weight, preferably not less than 85 % by weight, more preferably not less than 90 % by weight. Moreover, the concentration of the dialkyl carbonate in the high boiling point mixture $B_B$ can easily be made to be not less than 97 % by weight, preferably not less than 99 % by weight, more preferably not less than 99.9 % by weight, yet more preferably not less than 99.99 % by weight. Furthermore, the amount of the alcohol separated out as the main component of the low boiling point mixture $B_T$ is generally not less than 500 kg / hr, preferably not less than 1 ton / hr, more preferably not less than 2 ton / hr. The remainder of the low boiling point mixture $B_T$ is mostly the dialkyl carbonate, and hence the low boiling point mixture $B_T$ can be reused as aliphatic monohydric alcohol for reacting with the cyclic carbonate either as is or else after having been mixed with alcohol recovered from another process. This is one preferable embodiment of the present invention. In the case that the amount of recovered alcohol is insufficient, fresh aliphatic monohydric alcohol may be added.

The high boiling point mixture $B_B$ separated off in step (II) has the dialkyl carbonate as the main component thereof, and has a content of unreacted aliphatic monohydric alcohol of not more than 3 % by weight, preferably not more than 1 % by weight, more preferably not more than 0.1 % by weight, yet more preferably not more than 0.01 % by weight. Moreover, in a preferable embodiment of the present invention, the reaction is carried out using starting materials and catalyst not containing a halogen, and hence the produced dialkyl carbonate can be made to not contain a halogen at all. In the present invention, a high-purity dialkyl carbonate of concentration being not less than 97 % by weight, preferably not less than 99 % by weight, more preferably not less than 99.9 % by weight, yet more preferably not less than 99.99 % by weight, with a halogen content of not more than 0.1 ppm, preferably not more than 1 ppb (outside the detection limit for the ion chromatography), can thus be easily obtained.

The distillation conditions for the continuous multi-stage distillation column B used in step (II) vary depending on the form of the internal in the distillation column and the number of stages, the type, composition and amount of the low boiling point reaction mixture $A_T$ fed in, the purity of the dialkyl carbonate to be obtained through the separation, and so on. The column bottom temperature is generally a specified temperature in a range of from 150 to 250 °C. A more preferable temperature range is from 170 to 230 °C, yet more preferably from 180 to 220 °C. The column bottom pressure varies depending on the composition in the column and the column bottom temperature used, but in the present invention the continuous multi-stage distillation column B is generally operated under an applied pressure.

Moreover, the reflux ratio for the continuous multi-stage distillation column B is preferably in a range of from 0.5 to 5, more preferably from 0.8 to 3, yet more preferably from 1 to 2.5.

The material constituting the continuous multi-stage distillation column B used in step (II) is generally a metallic

material such as carbon steel or stainless steel. In terms of the quality of the dialkyl carbonate and the diol to be separated out, stainless steel is preferable.

EXAMPLES

Following is a more detailed description of the present invention through examples. However, the present invention is not limited to the following examples.

Example 1:

Step (I)

<Continuous multi-stage distillation column A>

A continuous multi-stage distillation column A as shown in FIG. 1 having $L_0$ = 3300 cm, $D_0$ = 300 cm, $L_0$ / $D_0$ = 11, $n_0$ = 60, $D_0$ / $d_{01}$ = 7.5, and $D_0$ / $d_{02}$ = 12 was used. The trays in the distillation column were sieve trays, each having a cross-sectional area per hole in a sieve portion thereof of approximately 1.3 $cm^2$ and a number of holes of approximately 180 to 320 / $m^2$. The aperture ratio of each of the trays was in a range of from 2.1 to 4.2%.

<Reactive distillation>

3.27 Ton / hr of ethylene carbonate in a liquid form was continuously introduced into the distillation column A from an inlet (3-a) provided at the $55^{th}$ stage from the bottom. 3.238 Ton / hr of methanol in a gaseous form (containing 8.96 % by weight of dimethyl carbonate) and 7.489 ton / hr of methanol in a liquid form (containing 6.66 % by weight of dimethyl carbonate) were respectively continuously introduced into the distillation column A from inlets (3-b and 3-c) provided at the $31^{st}$ stage from the bottom. The molar ratio of the starting materials introduced into the distillation column A was methanol / ethylene carbonate = 8.36.

The catalyst used was obtained by adding 4.8 ton of ethylene glycol to 2.5 ton of KOH (48 % by weight aqueous solution), heating to approximately 130 °C, gradually reducing the pressure, and carrying out heat treatment for approximately 3 hours at approximately 1300 Pa, so as to produce a homogeneous solution. This catalyst solution was continuously introduced into the distillation column A from an inlet (3-e) provided at the $54^{th}$ stage from the bottom (K concentration: 0.1 % by weight based on ethylene carbonate fed in). Reactive distillation was carried out continuously under conditions of a column bottom temperature of 98 °C, a column top pressure of approximately $1.118 \times 10^5$ Pa, and a reflux ratio of 0.42.

It was possible to attain stable steady state operation after 24 hours. A low boiling point reaction mixture $A_T$ continuously withdrawn from the top 1 of the column at 10.678 ton / hr contained 4.129 ton / hr of dimethyl carbonate, and 6.549 ton / hr of methanol. A liquid continuously withdrawn from the bottom 2 of the column at 3.382 ton / hr contained 2.356 ton / hr of ethylene glycol, 1.014 ton / hr of methanol, and 4 kg / hr of unreacted ethylene carbonate. Excluding the dimethyl carbonate contained in the starting material, the actual produced amount of dimethyl carbonate was 3.340 ton / hr, and excluding the ethylene glycol contained in the catalyst solution, the actual produced amount of ethylene glycol was 2.301 ton / hr. The ethylene carbonate conversion was 99.88%, the dimethyl carbonate selectivity was not less than 99.99%, and the ethylene glycol selectivity was not less than 99.99%.

Step (II)

<Continuous multi-stage distillation column B>

A continuous multi-stage distillation column B as shown in FIG. 2 having $L_1$ = 1600 cm, $D_1$ = 260 cm, $L_1$ / $D_1$ = 6.2, $n_1$ = 18, $L_2$ = 2700 cm, $D_2$ = 160 cm, $L_2$ / $D_2$ = 16.9, and $n_2$ = 58 was used. In this example, sieve trays were used as the internals in both the stripping section and the enrichment section (cross-sectional area per hole = approximately 1.3 $cm^2$, number of holes = approximately 300 to 440 / $m^2$).

<Separation by distillation of column top component $A_T$>

The column top component $A_T$ containing 4.129 ton / hr of dimethyl carbonate and 6.549 ton / hr of methanol obtained in step (I) was continuously fed into the continuous multi-stage distillation column B from an inlet (3-b). This inlet was provided between the trays in the $18^{th}$ and $19^{th}$ stages from the bottom of the continuous multi-stage distillation column B. The continuous multi-stage distillation column B was operated continuously with a column bottom temperature

of approximately 205 °C, a column bottom pressure of approximately 1.46 MPa, and a reflux ratio of approximately 1.8.

It was possible to attain stable steady state operation after 24 hours. A column top component $B_T$ continuously withdrawn from the top 1 of the continuous multi-stage distillation column B at 7.158 ton / hr contained 6.549 ton / hr of methanol and 0.509 ton / hr of dimethyl carbonate. The methanol concentration in the column top component $B_T$ was 91.49 % by weight. Moreover, a column bottom component $B_B$ continuously withdrawn from the bottom 2 of the continuous multi-stage distillation column B at 3.62 ton / hr contained not less than 99.99 % by weight of dimethyl carbonate (methanol content not more than 0.01 % by weight).

This means that of the dimethyl carbonate fed into the continuous multi-stage distillation column B, approximately 87.7% was obtained as high-purity dimethyl carbonate. Note that the column top component $B_T$ was fed as is into the reactive distillation column A, and thus used as some of the starting material for producing the dimethyl carbonate and the ethylene glycol.

Prolonged continuous operation was carried out under these conditions. After 500 hours, 2000 hours, 4000 hours, 5000 hours, and 6000 hours, the amounts of dimethyl carbonate obtained per hour were 3.62 ton, 3.62 ton, 3.62 ton, 3.62 ton, and 3.62 ton, and hence operation was very stable. The purity of the dimethyl carbonate obtained through the separation / purification was 99.99% in each case, and the halogen content was outside the detection limit, i.e. not more than 1 ppb.

Example 2:

Step (I)

Reactive distillation was carried out under the following conditions using the same continuous multi-stage distillation column A as in Example 1.

2.61 Ton / hr of ethylene carbonate in a liquid form was continuously introduced into the distillation column from the inlet (3-a) provided at the 55$^{th}$ stage from the bottom. 4.233 Ton / hr of methanol in a gaseous form (containing 2.41 % by weight of dimethyl carbonate) and 4.227 ton / hr of methanol in a liquid form (containing 1.46 % by weight of dimethyl carbonate) were respectively continuously introduced into the distillation column from the inlets (3-b and 3-c) provided at the 31$^{st}$ stage from the bottom. The molar ratio of the starting materials introduced into the distillation column was methanol / ethylene carbonate = 8.73. The catalyst was made to be the same as in Example 1, and was continuously fed into the distillation column. Reactive distillation was carried out continuously under conditions of a column bottom temperature of 93 °C, a column top pressure of approximately $1.046 \times 10^5$ Pa, and a reflux ratio of 0.48.

It was possible to attain stable steady state operation after 24 hours. A low boiling point reaction mixture $A_T$ continuously withdrawn from the top 1 of the column at 8.17 ton / hr contained 2.84 ton / hr of dimethyl carbonate, and 5.33 ton / hr of methanol. A liquid continuously withdrawn from the bottom 2 of the column at 2.937 ton / hr contained 1.865 ton / hr of ethylene glycol, 1.062 ton / hr of methanol, and 0.2 kg / hr of unreacted ethylene carbonate. Excluding the dimethyl carbonate contained in the starting material, the actual produced amount of dimethyl carbonate was 2.669 ton / hr, and excluding the ethylene glycol contained in the catalyst solution, the actual produced amount of ethylene glycol was 1.839 ton / hr. The ethylene carbonate conversion was 99.99%, the dimethyl carbonate selectivity was not less than 99.99%, and the ethylene glycol selectivity was not less than 99.99%.

Step (II)

Separation by distillation was carried out under the following conditions using the same continuous multi-stage distillation column B as in Example 1.

The column top component $A_T$ containing 2.84 ton / hr of dimethyl carbonate and 5.33 ton / hr of methanol obtained in step (I) was continuously fed into the continuous multi-stage distillation column B from an inlet (3-b). This inlet was provided between the trays in the 18$^{th}$ and 19$^{th}$ stages from the bottom of the continuous multi-stage distillation column B. The continuous multi-stage distillation column B was operated continuously with a column bottom temperature of approximately 205 °C, a column bottom pressure of approximately 1.46 MPa, and a reflux ratio of approximately 1.8.

It was possible to attain stable steady state operation after 24 hours. A column top component $B_T$ continuously withdrawn from the top 1 of the continuous multi-stage distillation column B at 5.76 ton / hr contained 5.33 ton / hr of methanol and 0.43 ton / hr of dimethyl carbonate. The methanol concentration in the column top component $B_T$ was 92.5 % by weight. Moreover, a column bottom component $B_B$ continuously withdrawn from the bottom 2 of the continuous multi-stage distillation column B at 2.41 ton / hr contained not less than 99.99 % by weight of dimethyl carbonate (methanol content not more than 0.01 % by weight).

This means that of the dimethyl carbonate fed into the continuous multi-stage distillation column B, approximately 84.9% was obtained as high-purity dimethyl carbonate. Note that the column top component $B_T$ was fed as is into the reactive distillation column A, and thus used as some of the starting material for producing the dimethyl carbonate and

ethylene glycol.

Prolonged continuous operation was carried out under these conditions. After 500 hours, 2000 hours, 4000 hours, 5000 hours, and 6000 hours, the amounts of dimethyl carbonate obtained per hour were 2.41 ton, 2.41 ton, 2.41 ton, 2.41 ton, and 2.41 ton, and hence operation was very stable. The purity of the dimethyl carbonate obtained through the separation / purification was 99.99% in each case, and the halogen content was outside the detection limit, i.e. not more than 1 ppb.

Example 3:

Step (I)

<Continuous multi-stage distillation column A>

A continuous multi-stage distillation column as shown in FIG. 1 having $L_0$ = 3300 cm, $D_0$ = 300 cm, $L_0$ / $D_0$ = 11, $n_0$ = 60, $D_0$ / $d_{01}$ = 7.5, and $D_0$ / $d_{02}$ = 12 was used. The trays in the distillation column were sieve trays, each having a cross-sectional area per hole in a sieve portion thereof of approximately 1.3 $cm^2$ and a number of holes of approximately 220 to 340 / $m^2$. The aperture ratio of each of the trays was in a range of from 2.5 to 4.5%.

<Reactive distillation>

3.773 Ton / hr of ethylene carbonate in a liquid form was continuously introduced into the distillation column from the inlet (3-a) provided at the 55$^{th}$ stage from the bottom. 3.736 Ton / hr of methanol in a gaseous form (containing 8.97 % by weight of dimethyl carbonate) and 8.641 ton / hr of methanol in a liquid form (containing 6.65 % by weight of dimethyl carbonate) were respectively continuously introduced into the distillation column from the inlets (3-b and 3-c) provided at the 31$^{st}$ stage from the bottom. The molar ratio of the starting materials introduced into the distillation column was methanol / ethylene carbonate = 8.73. The catalyst was made to be the same as in Example 1, and was continuously fed into the distillation column. Reactive distillation was carried out continuously under conditions of a column bottom temperature of 98 °C, a column top pressure of approximately $1.118 \times 10^5$ Pa, and a reflux ratio of 0.42.

It was possible to attain stable steady state operation after 24 hours. A low boiling point reaction mixture $A_T$ continuously withdrawn from the top 1 of the column at 12.32 ton / hr contained 4.764 ton / hr of dimethyl carbonate, and 7.556 ton / hr of methanol. A liquid continuously withdrawn from the bottom of the column at 3.902 ton / hr contained 2.718 ton / hr of ethylene glycol, 1.17 ton / hr of methanol, and 4.6 kg / hr of unreacted ethylene carbonate. Excluding the dimethyl carbonate contained in the starting material, the actual produced amount of dimethyl carbonate was 3.854 ton / hr, and excluding the ethylene glycol contained in the catalyst solution, the actual produced amount of ethylene glycol was 2.655 ton / hr. The ethylene carbonate conversion was 99.88%, the dimethyl carbonate selectivity was not less than 99.99%, and the ethylene glycol selectivity was not less than 99.99%.

Step (II)

Separation by distillation was carried out under the following conditions using the same continuous multi-stage distillation column B as in Example 1.

The column top component $A_T$ containing 4.764 ton / hr of dimethyl carbonate and 7.556 ton / hr of methanol obtained in step (I) was continuously fed into the continuous multi-stage distillation column B from an inlet (3-b). This inlet was provided between the trays in the 18$^{th}$ and 19$^{th}$ stages from the bottom of the continuous multi-stage distillation column B. The continuous multi-stage distillation column B was operated continuously with a column bottom temperature of approximately 205 °C, a column bottom pressure of approximately 1.46 MPa, and a reflux ratio of approximately 1.8.

It was possible to attain stable steady state operation after 24 hours. A column top component $B_T$ continuously withdrawn from the top 1 of the continuous multi-stage distillation column B at 8.231 ton / hr contained 7.556 ton / hr of methanol and 0.675 ton / hr of dimethyl carbonate. The methanol concentration in the column top component $B_T$ was 91.8 % by weight. Moreover, a column bottom component $B_B$ continuously withdrawn from the bottom 2 of the continuous multi-stage distillation column B at 4.089 ton / hr contained not less than 99.99 % by weight of dimethyl carbonate (methanol content not more than 0.01 % by weight).

This means that of the dimethyl carbonate fed into the continuous multi-stage distillation column B, approximately 85.8% was obtained as high-purity dimethyl carbonate. Note that the column top component $B_T$ was fed as is into the reactive distillation column A, and thus used as some of the starting material for producing the dimethyl carbonate and the ethylene glycol.

Prolonged continuous operation was carried out under these conditions. After 1000 hours, 2000 hours, 3000 hours, and 5000 hours, the amounts of dimethyl carbonate obtained per hour were 4.089 ton, 4.089 ton, 4.089 ton, and

4.089 ton, and hence operation was very stable. The purity of the dimethyl carbonate obtained through the separation / purification was 99.99% in each case, and the halogen content was outside the detection limit, i.e. not more than 1 ppb.

Example 4:

Step (I)

<Continuous multi-stage distillation column A>

A continuous multi-stage distillation column as shown in FIG. 1 having $L_0$ = 3300 cm, $D_0$ = 300 cm, $L_0$ / $D_0$ = 11, $n_0$ = 60, $D_0$ / $d_{01}$ = 7.5, and $D_0$ / $d_{02}$ = 12 was used. The trays in the distillation column were sieve trays, each having a cross-sectional area per hole in a sieve portion thereof of approximately 1.3 $cm^2$ and a number of holes of approximately 240 to 360 / $m^2$. The aperture ratio of each of the trays was in a range of from 3.0 to 5.0%.

<Reactive distillation>

7.546 Ton / hr of ethylene carbonate in a liquid form was continuously introduced into the distillation column from the inlet (3-a) provided at the 55th stage from the bottom. 7.742 Ton / hr of methanol in a gaseous form (containing 8.95 % by weight of dimethyl carbonate) and 17.282 ton / hr of methanol in a liquid form (containing 6.66 % by weight of dimethyl carbonate) were respectively continuously introduced into the distillation column from the inlets (3-b and 3-c) provided at the 31st stage from the bottom. The molar ratio of the starting materials introduced into the distillation column was methanol / ethylene carbonate = 8.36. The catalyst was made to be the same as in Example 1, and was continuously fed into the distillation column. Reactive distillation was carried out continuously under conditions of a column top temperature of 65 °C, a column top pressure of approximately $1.118\times10^5$ Pa, and a reflux ratio of 0.42.

It was possible to attain stable steady state operation after 24 hours. A low boiling point reaction mixture $A_T$ continuously withdrawn from the top 1 of the column at 24.64 ton / hr contained 9.527 ton / hr of dimethyl carbonate, and 15.114 ton / hr of methanol. A liquid continuously withdrawn from the bottom 2 of the column at 7.804 ton / hr contained 5.436 ton / hr of ethylene glycol, 2.34 ton / hr of methanol, and 23 kg / hr of unreacted ethylene carbonate. Excluding the dimethyl carbonate contained in the starting material, the actual amount produced of dimethyl carbonate was 7.708 ton / hr, and excluding the ethylene glycol contained in the catalyst solution, the actual produced amount of ethylene glycol was 5.31 ton / hr. The ethylene carbonate conversion was 99.7%, the dimethyl carbonate selectivity was not less than 99.99%, and the ethylene glycol selectivity was not less than 99.99%.

Step (II)

<Continuous multi-stage distillation column B>

A continuous multi-stage distillation column B as shown in FIG. 2 having $L_1$ = 1600 cm, $D_1$ = 260 cm, $L_1$ / $D_1$ = 6.2, $n_1$ = 18, $L_2$ = 2700 cm, $D_2$ = 160 cm, $L_2$ / $D_2$ = 16.9, and $n_2$ = 58 was used. In this example, sieve trays were used as the internal in both the stripping section and the enrichment section (cross-sectional area per hole = approximately 1.3 $cm^2$, number of holes = approximately 530 to 800 / $m^2$).

<Separation by distillation of column top component $A_T$>

The column top component $A_T$ containing 9.527 ton / hr of dimethyl carbonate and 15.114 ton / hr of methanol obtained in step (I) was continuously fed into the continuous multi-stage distillation column B from an inlet (3-b). This inlet was provided between the trays in the 18th and 19th stages from the bottom of the continuous multi-stage distillation column B. The continuous multi-stage distillation column B was operated continuously with a column bottom temperature of approximately 205 °C, a column bottom pressure of approximately 1.46 MPa, and a reflux ratio of approximately 1.8.

It was possible to attain stable steady state operation after 24 hours. A column top component $B_T$ continuously withdrawn from the top 1 of the continuous multi-stage distillation column B at 16.572 ton / hr contained 15.114 ton / hr of methanol and 1.458 ton / hr of dimethyl carbonate. The methanol concentration in the column top component $B_T$ was 91.2 % by weight. Moreover, a column bottom component $B_B$ continuously withdrawn from the bottom 2 of the continuous multi-stage distillation column B at 8.069 ton / hr contained not less than 99.99 % by weight of dimethyl carbonate (methanol content not more than 0.01 % by weight).

This means that of the dimethyl carbonate fed into the continuous multi-stage distillation column B, approximately 84.7% was obtained as high-purity dimethyl carbonate. Note that the column top component $B_T$ was fed as is into the reactive distillation column A, and thus used as some of the starting material for producing the dimethyl carbonate and

the ethylene glycol.

Prolonged continuous operation was carried out under these conditions. After 1000 hours, the amount of dimethyl carbonate obtained per hour was 8.069 ton, and hence operation was very stable. The purity of the dimethyl carbonate obtained through the separation/purification was 99.99%, and the halogen content was outside the detection limit, i.e. not more than 1 ppb.

Industrial Applicability

According to the present invention, it has been discovered that a dialkyl carbonate of high purity (e.g. not less than 97%, preferably not less than 99%, more preferably not less than 99.9%, yet more preferably not less than 99.99%) and a diol can be produced each with a high selectivity of not less than 95%, preferably not less than 97%, more preferably not less than 99%, on an industrial scale of not less than 2 ton / hr, preferably not less than 3 ton / hr, more preferably not less than 4 ton / hr, for the dialkyl carbonate, and not less than 1.3 ton / hr, preferably not less than 1.95 ton / hr, more preferably not less than 2.6 ton / hr, for the diol, with a high yield stably for a prolonged period of time of not less than 1000 hours, preferably not less than 3000 hours, more preferably not less than 5000 hours, from a cyclic carbonate and an aliphatic monohydric alcohol.

**Claims**

**1.** An industrial process for the production of a dialkyl carbonate and a diol in which the dialkyl carbonate and the diol are continuously produced from a cyclic carbonate and an aliphatic monohydric alcohol, comprising the steps of:

(I) continuously producing a dialkyl carbonate and a diol through a reactive distillation system of continuously feeding the cyclic carbonate and the aliphatic monohydric alcohol into a continuous multi-stage distillation column A in which a catalyst is present, carrying out reaction and distillation simultaneously in said column, continuously withdrawing a low boiling point reaction mixture $A_T$ containing the produced dialkyl carbonate and unreacted aliphatic monohydric alcohol from an upper portion of the column A in a gaseous form, and continuously withdrawing a high boiling point reaction mixture $A_B$ containing the diol from a lower portion of the column A in a liquid form; and

(II) continuously feeding the low boiling point reaction mixture $A_T$ containing the dialkyl carbonate and the aliphatic monohydric alcohol continuously withdrawn from the upper portion of said continuous multi-stage distillation column A into a continuous multi-stage distillation column B, and continuously separating and purifying into a column top component $B_T$ having the aliphatic monohydric alcohol as a main component thereof and a column bottom component $B_B$ having the dialkyl carbonate as a main component thereof, wherein:

(a) said continuous multi-stage distillation column A is a tray column having a cylindrical trunk portion having a length $L_0$ (cm) and an inside diameter $D_0$ (cm) and having thereinside a tray with a number of stages no, the tray having a plurality of holes therein, and further having a gas outlet having an inside diameter $d_{01}$ (cm) at a top of the column or in an upper portion of the column near to the top, a liquid outlet having an inside diameter $d_{02}$ (cm) at a bottom of the column or in a lower portion of the column near to the bottom, at least one first inlet provided in the upper portion and / or a middle portion of the column below the gas outlet, and at least one second inlet provided in the middle portion and / or the lower portion of the column above the liquid outlet, wherein $L_0$, $D_0$, $n_0$, $d_{01}$ and $d_{02}$ satisfy the following formulae (1) to (6):

$$2100 \leq L_0 \leq 8000 \qquad (1)$$

$$180 \leq D_0 \leq 2000 \qquad (2)$$

$$4 \leq L_0 / D_0 \leq 40 \qquad (3)$$

$$20 \leq n_0 \leq 120 \quad (4)$$

$$3 \leq D_0 / d_{01} \leq 20 \quad (5)$$

$$5 \leq D_0 / d_{02} \leq 30 \quad (6);$$

and
(b) said continuous multi-stage distillation column B is a distillation column comprising a stripping section having a length $L_1$ (cm), an inside diameter $D_1$ (cm) and an internal with a number of stages $n_1$ thereinside, and an enrichment section having a length $L_2$ (cm), an inside diameter $D_2$ (cm) and an internal with a number of stages $n_2$ thereinside, wherein $L_1$, $D_1$, $n_1$, $L_2$, $D_2$, and $n_2$ satisfy the following formulae (7) to (14):

$$500 \leq L_1 \leq 3000 \quad (7)$$

$$100 \leq D_1 \leq 1000 \quad (8)$$

$$2 \leq L_1 / D_1 \leq 30 \quad (9)$$

$$10 \leq n_1 \leq 40 \quad (10)$$

$$700 \leq L_2 \leq 5000 \quad (11)$$

$$50 \leq D_2 \leq 800 \quad (12)$$

$$10 \leq L_2 / D_2 \leq 50 \quad (13)$$

$$35 \leq n_2 \leq 100 \quad (14).$$

**2.** The process according to Claim 1, wherein a produced amount of the dialkyl carbonate is not less than 2 ton / hr.

**3.** The process according to Claim 1 or 2, wherein a produced amount of the diol is not less than 1.3 ton / hr.

**4.** The process according to any one of Claims 1 to 3, wherein said $d_{01}$ and said $d_{02}$ satisfy the following formula (15);

$$1 \leq d_{01} / d_{02} \leq 5 \qquad (15).$$

**5.** The process according to any one of Claims 1 to 4, wherein $L_0$, $D_0$, $L_0 / D_0$, $n_0$, $D_0 / d_{01}$, and $D_0 / d_{02}$ for said continuous multi-stage distillation column A satisfy respectively $2300 \leq L_0 \leq 6000$, $200 \leq D_0 \leq 1000$, $5 \leq L_0 / D_0 \leq 30$, $30 \leq n_0 \leq 100$, $4 \leq D_0 / d_{01} \leq 15$, and $7 \leq D_0 / d_{02} \leq 25$.

**6.** The process according to any one of Claims 1 to 5, wherein $L_0$, $D_0$, $L_0 / D_0$, no, Do / $d_{01}$, and $D_0 / d_{02}$ for said continuous multi-stage distillation column A satisfy respectively $2500 \leq L_0 \leq 5000$, $210 \leq D_0 \leq 800$, $7 \leq L_0 / D_0 \leq 20$, $40 \leq n_0 \leq 90$, $5 \leq D_0 / d_{01} \leq 13$, and $9 \leq D_0 / d_{02} \leq 20$.

**7.** The process according to any one of Claims 1 to 6, wherein the tray in said continuous multi-stage distillation column A is a sieve tray.

**8.** The process according to Claim 7, wherein said sieve tray in said continuous multi-stage distillation column A has 100 to 1000 holes / $m^2$ in a sieve portion thereof.

**9.** The process according to Claim 7 or 8, wherein a cross-sectional area per hole of said sieve tray in said continuous multi-stage distillation column A is in a range of from 0.5 to 5 $cm^2$.

**10.** The process according to any one of Claims 7 to 9, wherein an aperture ratio of said sieve tray in said continuous multi-stage distillation column A is in a range of from 1.5 to 15%.

**11.** The process according to any one of Claims 1 to 10, wherein $L_1$, $D_1$, $L_1 / D_1$, $n_1$, $L_2$, $D_2$, $L_2 / D_2$, and $n_2$ for said continuous multi-stage distillation column B satisfy $800 \leq L_1 \leq 2500$, $120 \leq D_1 \leq 800$, $5 \leq L_1 / D_1 \leq 20$, $13 \leq n_1 \leq 25$, $1500 \leq L_2 \leq 3500$, $70 \leq D_2 \leq 600$, $15 \leq L_2 / D_2 \leq 30$, $40 \leq n_2 \leq 70$, $L_1 \leq L_2$, and $D_2 \leq D_1$.

**12.** The process according to any one of Claims 1 to 11, wherein the internal in each of the stripping section and the enrichment section of said continuous multi-stage distillation column B is a tray and / or a packing.

**13.** The process according to Claim 12, wherein the internal in each of the stripping section and the enrichment section of said continuous multi-stage distillation column B is the tray.

**14.** The process according to Claim 13, wherein said tray in said continuous multi-stage distillation column B is a sieve tray.

**15.** The process according to Claim 14, wherein said sieve tray in said continuous multi-stage distillation column B has 150 to 1200 holes / $m^2$ in a sieve portion thereof, and a cross-sectional area per hole in a range of from 0.5 to 5 $cm^2$.

**16.** The process according to any one of Claims 1 to 15, wherein a concentration of the dialkyl carbonate in said column bottom component $B_B$ said the continuous multi-stage distillation column B is not less than 97 % by weight based on 100 % by weight of said column bottom component.

**17.** The process according to any one of Claims 1 to 15, wherein the concentration of the dialkyl carbonate in said column bottom component $B_B$ from said continuous multi-stage distillation column B is not less than 99 % by weight based on 100 % by weight of said column bottom component.

**18.** The process according to any one of Claims 1 to 17, wherein said column top component $B_T$ from said continuous multi-stage distillation column B is recycled as a starting material for producing a dialkyl carbonate and a diol.

**19.** The process according to any one of Claims 1 to 18, wherein the cyclic carbonate comprises ethylene carbonate and / or propylene carbonate, the aliphatic monohydric alcohol comprises methanol and / or ethanol, the dialkyl carbonate to be produced comprises dimethyl carbonate and / or diethyl carbonate, and the diol to be produced comprises ethylene glycol and / or propylene glycol.

**20.** A dialkyl carbonate separated out by the process according to any one of Claims 1 to 19, which comprises a halogen content of not more than 0.1 ppm.

**21.** A dialkyl carbonate separated out by the process according to any one of Claims 1 to 19, which comprises a halogen content of not more than 1 ppb.

**22.** The dialkyl carbonate according to Claim 20 or 21, which is a high-purity dialkyl carbonate having an aliphatic monohydric alcohol content of not more than 0.1 % by weight.

# FIG.1

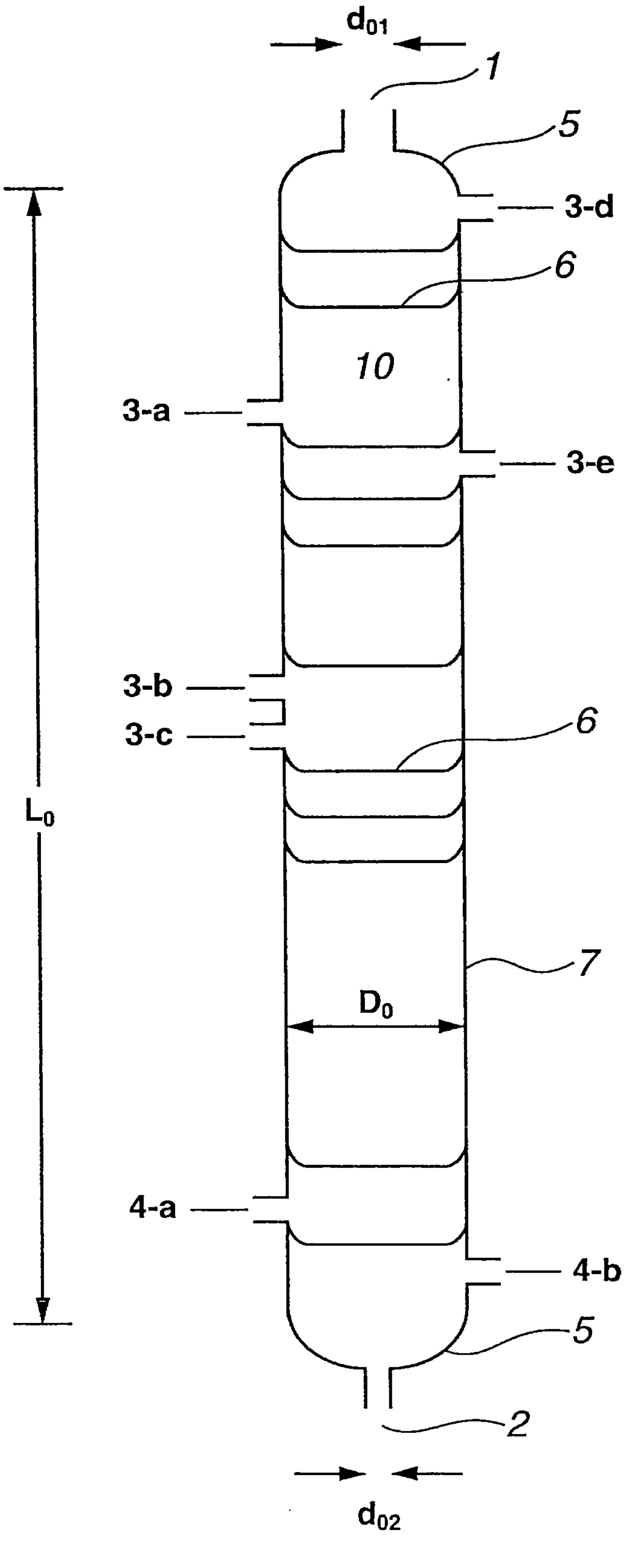

# FIG.2

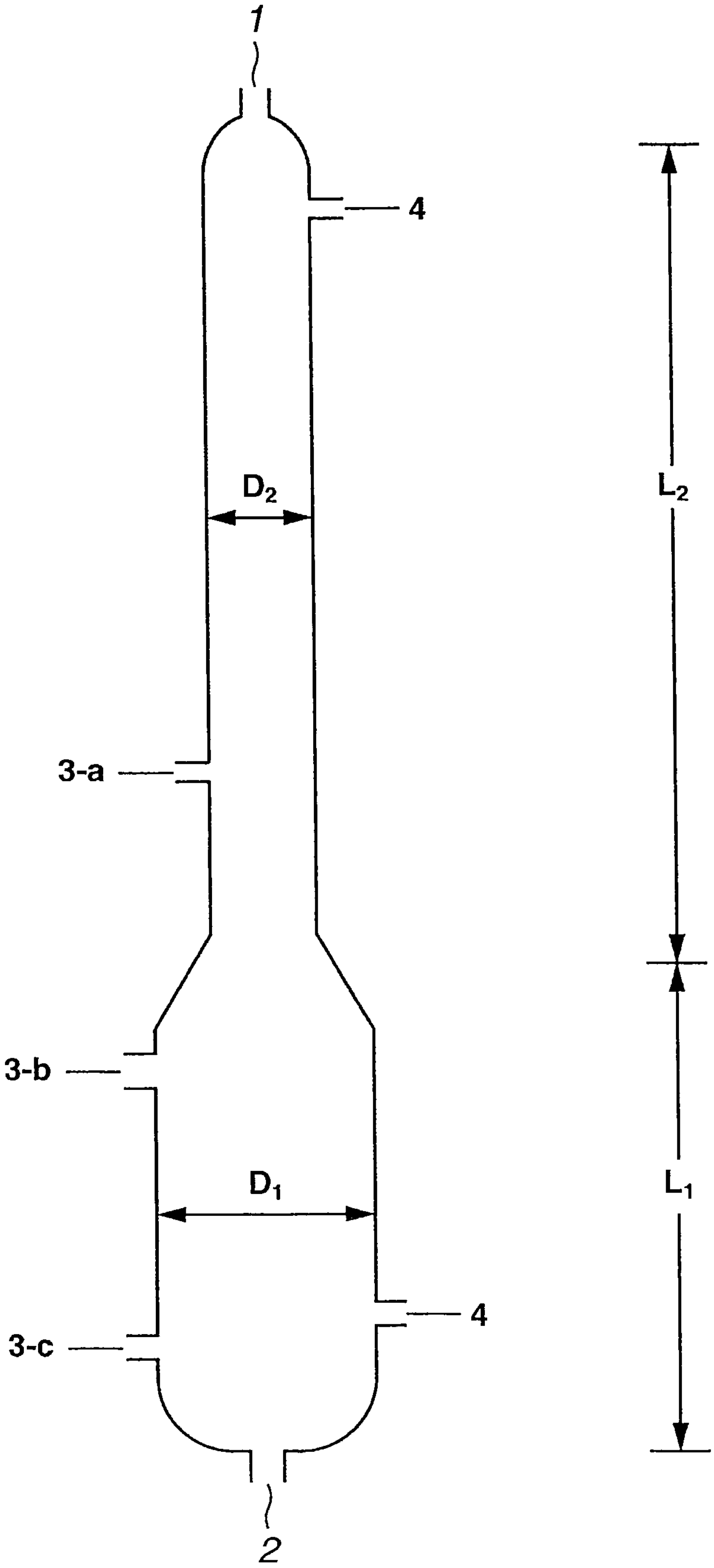

## INTERNATIONAL SEARCH REPORT

International application No.
PCT/JP2006/325354

A. CLASSIFICATION OF SUBJECT MATTER
*C07C27/02*(2006.01)i, *B01D3/14*(2006.01)i, *B01D3/22*(2006.01)i, *B01D3/32*(2006.01)i, *C07C29/128*(2006.01)i, *C07C31/20*(2006.01)i, *C07C68/06*(2006.01)i, *C07C68/08*(2006.01)i, *C07C69/96*(2006.01)i, *C07B61/00*(2006.01)n
According to International Patent Classification (IPC) or to both national classification and IPC

B. FIELDS SEARCHED
Minimum documentation searched (classification system followed by classification symbols)
C07C27/02, B01D3/14, B01D3/22, B01D3/32, C07C29/128, C07C31/20, C07C68/06, C07C68/08, C07C69/96, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2002-371037 A (Mitsubishi Chemical Corp.), 26 December, 2002 (26.12.02), Claims; examples (Family: none) | 20-22<br>1-19 |
| X<br>A | JP 6-228026 A (Mitsubishi Gas Chemical Co., Inc.), 16 August, 1994 (16.08.94), Claims; Par. No. [0002]; example 14 (Family: none) | 20-22<br>1-19 |
| X<br>A | JP 2000-281630 A (Mitsubishi Chemical Corp.), 10 October, 2000 (10.10.00), Claims; Par. Nos. [0010] to [0011]; examples (Family: none) | 20-22<br>1-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>31 January, 2007 (31.01.07) | Date of mailing of the international search report<br>13 February, 2007 (13.02.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/325354

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 7-25830 A (Daicel Chemical Industries, Ltd.),<br>27 January, 1995 (27.01.95),<br>Claims; examples<br>(Family: none) | 20-22<br>1-19 |
| X<br>A | JP 6-196464 A (Kabushiki Kaisha Sanshiru),<br>15 July, 1994 (15.07.94),<br>Claims; Par. No. [0010]<br>(Family: none) | 20-22<br>1-19 |
| A | WO 00/51954 A1 (Asahi Chemical Industry Co., Ltd.),<br>08 September, 2000 (08.09.00),<br>& EP 1174406 A1 & US 6479689 B1 | 1-22 |
| A | JP 9-183744 A (Asahi Chemical Industry Co., Ltd.),<br>15 July, 1997 (15.07.97),<br>(Family: none) | 1-22 |
| A | JP 2003-342209 A (Mitsubishi Chemical Corp.),<br>03 December, 2003 (03.12.03),<br>(Family: none) | 1-22 |
| P,A | WO 2005/123638 A1 (Asahi Kasei Chemicals Corp.),<br>29 December, 2005 (29.12.05),<br>(Family: none) | 1-22 |
| P,A | WO 2006/001256 A1 (Asahi Kasei Chemicals Corp.),<br>05 January, 2006 (05.01.06),<br>(Family: none) | 1-22 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3642858 A
- JP S5448715 B
- US 4181676 A
- JP S5463023 B
- JP S54148726 B
- JP S51122025 B
- US 4062884 A
- JP S5448716 B
- US 4307032 A
- US 3803201 A
- JP 63041432 A
- US 4661609 A
- US 4734518 A
- JP S63238043 B
- JP S6431737 B
- US 4691041 A
- JP S6341432 B
- JP H4198141 B
- JP H4230243 B
- JP H9176061 B
- JP H9183744 B
- JP H9194435 B
- WO 9723445 A
- EP 0889025 A
- US 5847189 A
- WO 9964382 A
- EP 1086940 A
- US 6346638 B
- WO 0051954 A
- EP 1174406 A
- US 6479689 B
- JP 2002308804 A
- JP 2004131394 A
- JP H5213830 B
- JP 0530615 A
- US 5231212 A
- JP H69507 A
- EP 0569812 A
- US 5359118 A
- JP 2003119168 A
- WO 03006418 A
- JP 2003300936 A
- JP 2003342209 A